# EUROPEAN PATENT SPECIFICATION

(11) **EP 1 343 780 B1**
(45) Date of publication and mention of the grant of the patent: **20.09.2006**
(21) Application number: 01271369.9
(22) Date of filing: 19.12.2001
(51) Int. Cl.: C07D 401/14, C07D 471/04, A61K 31/495, A61P 31/04, C07D 221/00

(54) **PIPERAZINE DERIVATIVES FOR TREATMENT OF BACTERIAL INFECTIONS**
PIPERAZINDERIVATE ZUR BEHANDLUNG BAKTERIELLER INFEKTIONEN
DERIVES DE LA PIPERAZINE UTILISES POUR LE TRAITEMENT D'INFECTIONS BACTERIENNES

(30) Priority: 20.12.2000 GB 0031088
(43) Date of publication of application: 17.09.2003
(73) Proprietor: SMITHKLINE BEECHAM PLC, Brentford, Middlesex TW8 9GS (GB)
(72) Inventor: DARTOIS, C.G.Y., CIP, CN925.1, Brentford, Middlesex, TW8 9GS (GB); MARKWELL, Roger Edward, GlaxoSmithKline, Harlow, Essex CM19 5AW (GB); MORVAN, Marcel, CIP CN925.1, Brentford Middlesex, TW8 9GS (GB); NADLER, G.M.G., CIP, CN925.1, Brentford, Middlesex, TW8 9GS (GB); PEARSON, Neil David, GlaxoSmithKline, Harlow, Essex CM19 5AW (GB)
(74) Representative: Valentine, Jill Barbara
(86) International application number: PCT/GB2001/005653
(87) International publication number: WO 2002/050061

(56) References cited:
- WO-A-00/21948
- WO-A-00/21952
- WO-A-99/37635
- BE-A- 645 602

## Description

This invention relates to novel compounds, compositions containing them and their use as antibacterials.

WO99/37635, WO00/21948 and WO00/21952 disclose piperidine derivatives having antibacterial activity.

EP0579263, EP0742207, JP2169569 and EP0296560, generically disclose piperazine compounds as acetylcholinesterase inhibitors and sigma receptor antagonists

WO92/17475, WO98/02438, WO97/03069 and WO96/39145 disclose certain bicyclic heteroaromatic compounds having cholinesterase inhibitor, protein tyrosine kinase inhibitor, cell proliferation inhibitor and human epidermal growth factor receptor type 2 inhibitor activity.

JP7179407 discloses bicyclic heteroaromatic compounds having GPIIb/IIIa inhibitory activity

WO97/17973 discloses piperazine derivatives having hemoregulatory activities.

WO99/05096 discloses naphthamidine compounds having urokinase inhibitory activity.

BE645602 discloses piperazine derivatives having antimalerial, anthelmintic and amoebicide activity.

This invention provides a compound of formula (I) or a pharmaceutically acceptable salt and/or N-oxide thereof: 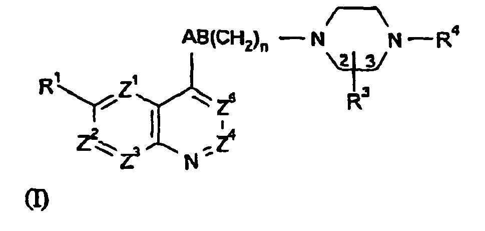 wherein:
one of Z¹, Z², Z³, Z⁴ and Z⁵ is N, one is CR^{1a} and the remainder are CH, or one of Z¹, Z², Z³, Z⁴ and Z⁵ is CR^{1a} and the remainder are CH;
R¹ and R^{1a} are independently selected from hydrogen; hydroxy; (C₁₋₆) alkoxy optionally substituted by (C₁₋₆)alkoxy, amino, piperidyl, guanidino or amidino any of which is optionally N-substituted by one or two (C₁₋₆)alkyl, acyl or (C₁₋₆) alkylsulphonyl groups, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy-substituted (C₁₋₆) alkyl; halogen; (C₁₋₆)alkyl; (C₁₋₆)alkylthio; trifluoromethyl; nitro; azido; acyl; acyloxy; acylthio; (C₁₋₆)alkylsulphonyl; (C₁₋₆)alkylsulphoxide; arylsulphonyl; arylsulphoxide or an amino, piperidyl, guanidino or amidino group optionally N-substituted by one or two (C₁₋₆)alkyl, acyl or (C₁₋₆)alkylsulphonyl groups,
   or when Z⁵ is CR^{1a}, R^{1a} may instead be cyano, hydroxymethyl or carboxy,
   provided that when none of Z¹, Z², Z³, Z⁴ and Z⁵ is N, then R¹ is not hydrogen;
R³ is hydrogen; or
R³ is in the 2- or 3-position and is:
   carboxy; (C₁₋₆)alkoxycarbonyl; aminocarbonyl wherein the amino group is optionally substituted by hydroxy, (C₁₋₆)alkyl, hydroxy(C₁₋₆)alkyl, aminocarbonyl(C₁₋₆)alkyl, (C₂₋₆)alkenyl, (C₁₋₆)alkylsulphonyl, trifluoromethylsulphonyl, (C₂₋₆)alkenylsulphonyl, (C₁₋₆)alkoxycarbonyl, (C₁₋₆)alkylcarbonyl, (C₂₋₆)alkenyloxycarbonyl or (C₂₋₆) alkenylcarbonyl and optionally further substituted by (C₁₋₆)alkyl, hydroxy(C₁₋₆)alkyl, aminocarbonyl(C₁₋₆)alkyl or (C₂₋₆)alkenyl; cyano; tetrazolyl; 2-oxo-oxazolidinyl optionally substituted by R¹⁰; 3-hydroxy-3-cyclobutene-1,2-dione-4-yl; 2,4-thiazolidinedione-5-yl; tetrazol-5-ylaminocarbonyl; 1,2,4-triazol-5-yl optionally substituted by R¹⁰; or 5-oxo-1,2,4-oxadiazol-3-yl; or
   (C₁₋₄)alkyl or ethenyl optionally substituted with any of the groups listed above for R³ and/or 0 to 2 groups R¹² independently selected from:
      halogen; (C₁₋₆)alkylthio; trifluoromethyl; (C₁₋₆)alkoxycarbonyl; (C₁₋₆) alkylcarbonyl; (C₂₋₆)alkenyloxycarbonyl; (C₂₋₆)alkenylcarbonyl; hydroxy optionally substituted by (C₁₋₆)alkyl, (C₂₋₆)alkenyl, (C₁₋₆)alkoxycarbonyl, (C₁₋₆)alkylcarbonyl, (C₂₋₆)alkenyloxycarbonyl, (C₂₋₆)alkenylcarbonyl or aminocarbonyl wherein the amino group is optionally substituted by (C₁₋₆)alkyl, (C₂₋₆)alkenyl, (C₁₋₆)alkylcarbonyl or (C₂₋₆)alkenylcarbonyl; amino optionally mono- or disubstituted by (C₁₋₆) alkoxycarbonyl, (C₁₋₆)alkylcarbonyl, (C₂₋₆)alkenyloxycarbonyl, (C₂₋₆) alkenylcarbonyl, (C₁₋₆)alkyl, (C₂₋₆)alkenyl, (C₁₋₆)alkylsulphonyl, (C₂₋₆) alkenylsulphonyl or aminocarbonyl wherein the amino group is optionally substituted by (C₁₋₆)alkyl or (C₂₋₆)alkenyl; aminocarbonyl wherein the amino group is optionally substituted by (C₁₋₆)alkyl, hydroxy(C₁₋₆)alkyl, aminocarbonyl(C₁₋₆)alkyl, (C₂₋₆) alkenyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆) alkyl, aminocarbonyl(C₁₋₆)alkyl or (C₂₋₆)alkenyl; oxo; (C₁₋₆)alkylsulphonyl; (C₂₋₆) alkenylsulphonyl; or (C₁₋₆)aminosulphonyl wherein the amino group is optionally substituted by (C₁₋₆)alkyl or (C₂₋₆)alkenyl;
      in addition when R³ is disubstituted with a hydroxy or amino containing substituent and a carboxy containing substituent these may optionally together form a cyclic ester or amide linkage, respectively;
R¹⁰ is selected from (C₁₋₄)alkyl; (C₂₋₄)alkenyl and aryl any of which may be optionally substituted by a group R¹² as defined above; carboxy; and aminocarbonyl wherein the amino group is optionally substituted by hydroxy, (C₁₋₆)alkyl, (C₂₋₆)alkenyl, (C₁₋₆) alkylsulphonyl, trifluoromethylsulphonyl, (C₂₋₆)alkenylsulphonyl, (C₁₋₆) alkoxycarbonyl, (C₁₋₆)alkylcarbonyl, (C₂₋₆)alkenyloxycarbonyl or (C₂₋₆) alkenylcarbonyl;
R⁴ is a group -U-V-R⁵ where R⁵ is an optionally substituted bicyclic carbocyclic or heterocyclic ring system (A): 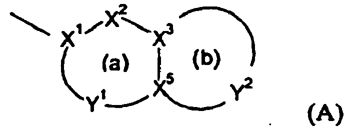 containing up to four heteroatoms in each ring in which
   at least one of rings (a) and (b) is aromatic;
   X¹ is C or N when part of an aromatic ring or CR¹⁴ or N when part of a non-aromatic ring;
   X² is N, NR¹³, O, S(O)ₓ, CO or CR¹⁴ when part of an aromatic or non-aromatic ring or may in addition be CR¹⁴R¹⁵ when part of a non-aromatic ring;
   X³ and X⁵ are independently N or C;
   Y¹ is a 0 to 4 atom linker group each atom of which is independently selected from N, NR¹³, O, S(O)ₓ, CO and CR¹⁴ when part of an aromatic or non-aromatic ring or may additionally be CR¹⁴R¹⁵ when part of a non-aromatic ring,
   Y² is a 2 to 6 atom linker group, each atom of Y² being independently selected from N, NR¹³, O, S(O)ₓ, CO and CR¹⁴ when part of an aromatic or non-aromatic ring or may additionally be CR¹⁴R¹⁵ when part of a non-romatic ring; each of R¹⁴ and R¹⁵ is independently selected from: H; (C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄) alkenylcarbonyl; (C₁₋₄)alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted as for corresponding substituents in R³; (C₁₋₄) alkylsulphonyl; (C₂₋₄)alkenylsulphonyl; or aminosulphonyl wherein the amino group is optionally substituted by (C₁₋₄)alkyl or (C₂₋₄)alkenyl; aryl; aryl(C₁₋₄)alkyl; and aryl(C₁₋₄) alkoxy;
   each R¹³ is independently H; trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; aryl; aryl (C₁₋₄)alkyl; (C₁₋₄)alkoxycarbonyl; (C₁₋₄)alkylcarbonyl; formyl; (C₁₋₆) alkylsulphonyl; or aminocarbonyl wherein the amino group is optionally substituted by (C₁₋₄)alkoxycarbonyl, (C₁₋₄)alkylcarbonyl, (C₂₋₄)alkenyloxycarbonyl, (C₂₋₄) alkenylcarbonyl, (C₁₋₄)alkyl or (C₂₋₄)alkenyl and optionally further substituted by (C₁₋₄)alkyl or (C₂₋₄)alkenyl;
U is selected from CO, SO₂ and CH₂ and V is CR¹⁷R¹⁸ or U is CH₂ and V is CO or SO₂;
R¹⁷ and R¹⁸ are independently selected from hydrogen; hydroxy optionally substituted by (C₁₋₆)alkyl, (C₂₋₆)alkenyl, (C₁₋₆)alkoxycarbonyl, (C₁₋₆)alkylcarbonyl, (C₂₋₆) alkenyloxycarbonyl; (C₂₋₆)alkenylcarbonyl or aminocarbonyl wherein the amino group is optionally substituted by (C₁₋₆)alkyl, (C₂₋₆)alkenyl, (C₁₋₆)alkylcarbonyl or (C₂₋₆) alkenylcarbonyl; and amino optionally mono- or disubstituted by (C₁₋₆) alkoxycarbonyl, (C₁₋₆)alkylcarbonyl, (C₂₋₆)alkenyloxycarbonyl, (C₂₋₆) alkenylcarbonyl, (C₁₋₆)alkyl, (C₂₋₆)alkenyl, (C₁₋₆)alkylsulphonyl, (C₂₋₆) alkenylsulphonyl or aminocarbonyl wherein the amino group is optionally substituted by (C₁₋₆)alkyl or (C₂₋₆)alkenyl;
n is 0 and AB is NR¹¹CO, CO-CR⁸R⁹, CR⁶R⁷-CO, NR¹¹SO₂, CR⁶R⁷-SO₂ or CR⁶R⁷-CR⁸R⁹, provided that R⁸ and R⁹ are not optionally substituted hydroxy or amino and R⁶ and R⁸ do not represent a bond:
   or n is 1 and AB is NR¹¹CO, CO-CR⁸R⁹, CR⁶R⁷-CO, NR¹¹SO₂, CONR¹¹, CR⁶R⁷⁻CR⁸R⁹, O-CR⁸R⁹ or NR¹¹-CR⁸R⁹;
and wherein:
each of R⁶, R⁷, R⁸ and R⁹ is independently selected from: H; (C₁₋₆)alkoxy; (C₁₋₆) alkylthio; halo; trifluoromethyl; azido; (C₁₋₆)alkyl; (C₂₋₆)alkenyl; (C₁₋₆) alkoxycarbonyl; (C₁₋₆)alkylcarbonyl; (C₂₋₆)alkenyloxycarbonyl; (C₂₋₆) alkenylcarbonyl; hydroxy, amino or aminocarbonyl optionally substituted as for corresponding substituents in R³; (C₁₋₆)alkylsulphonyl; (C₂₋₆)alkenylsulphonyl; or (C₁₋₆) aminosulphonyl wherein the amino group is optionally substituted by (C₁₋₆)alkyl or (C₂₋₆)alkenyl;
or R⁶ and R⁸ together represent a bond and R⁷ and R⁹ are as above defined;
and each R¹¹ is independently H; trifluoromethyl; (C₁₋₆)alkyl; (C₂₋₆)alkenyl; (C₁₋₆) alkoxycarbonyl; (C₁₋₆)alkylcarbonyl; or aminocarbonyl wherein the amino group is optionally substituted by (C₁₋₆)alkoxycarbonyl, (C₁₋₆)alkylcarbonyl, (C₂₋₆) alkenyloxycarbonyl, (C₂₋₆)alkenylcaxbonyl, (C₁₋₆)alkyl or (C₂₋₆)alkenyl and optionally further substituted by (C₁₋₆)alkyl or (C₂₋₆)alkenyl;
or where one of R³ and R⁶, R⁷, R⁸ or R⁹ contains a carboxy group and the other contains a hydroxy or amino group they may together form a cyclic ester or amide linkage;
wherein
'heterocyclic' is an aromatic or non-aromatic, single or fused, ring containing up to four hetero-atoms in each ring selected from oxygen, nitrogen and sulphur, and having from 4 to 7 ring atoms, which rings may be unsubstituted or C-substituted by up to three groups selected from (C₁₋₄)alkylthio; halo; carboxy(C₁₋₄)alkyl; halo(C₁₋₄)alkoxy; halo(C₁₋₄) alkyl; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄)alkenylcarbonyl; (C₁₋₄)alkylcarbonyloxy; (C₁₋₄) alkoxycarbonyl(C₁₋₄)alkyl; hydroxy; hydroxy(C₁₋₄)alkyl; mercapto(C₁₋₄)alkyl, (C₁₋₄) alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄) alkenyl; optionally substituted aryl, aryl(C₁₋₄)alkyl or aryl(C₁₋₄)alkoxy and oxo, and wherein any amino group forming part of a single or fused non-aromatic heterocyclic ring as defined above is optionally substituted by trifluoromethyl; (C₁₋₄)alkyl optionally substituted by hydroxy, (C₁₋₆)alkoxy, (C₁₋₆)alkylthio, halo or trifluoromethyl; (C₂₋₄) alkenyl; aryl; aryl (C₁₋₄)alkyl; (C₁₋₄)alkoxycarbonyl; (C₁₋₄)alkylcarbonyl; formyl; (C₁₋₆)alkylsulphonyl; or aminocarbonyl wherein the amino group is optionally substituted by (C₁₋₄)alkoxycarbonyl, (C₁₋₄)alkylcarbonyl, (C₂₋₄)alkenyloxycarbonyl, (C₂₋₄)alkenylcarbonyl, (C₁₋₄)alkyl or (C₂₋₄)alkenyl and optionally further substituted by (C₁₋₄)alkyl or (C₂₋₄)alkenyl;
'aryl' is phenyl or naphthyl optionally substituted with up to five groups selected from (C₁₋₄)alkylthio; halo; carboxy(C₁₋₄)alkyl; helo(C₁₋₄)alkoxy; halo(C₁₋₄)alkyl; (C₁₋₄) alkyl; (C₂₋₄)alkenyl; (C₁₋₄)alkoxycarbonyl; formyl; (C₁₋₄)alkylcarbonyl; (C₂₋₄) alkenyloxycarbonyl; (C₂₋₄)alkenylcarbonyl; (C₁₋₄)alkylcarbonyloxy; (C₁₋₄) alkoxycarbonyl(C₁₋₄)alkyl; hydroxy; hydroxy(C₁₋₄)alkyl; mercapto(C₁₋₄)alkyl; (C₁₋₄) alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄) alkenyl; phenyl, phenyl(C₁₋₄)alkyl and phenyl(C₁₋₄)alkoxy;
'acyl' is (C₁₋₆)alkoxycarbonyl, formyl or (C₁₋₆) alkylcarbonyl.

The invention also provides the use of a compound of formula (I) or a pharmaceutically acceptable salt and/or N-oxide thereof in the manufacture of a medicament for use in the treatment of bacterial infections in mammals.

The invention also provides a pharmaceutical composition for use in the treatment of bacterial infections in mammals comprising a compound of formula (I), or a pharmaceutically acceptable salt and/or N-oxide thereof, and a pharmaceutically acceptable carrier.

In one aspect, when (a) and (b) are both aromatic, -U-V- is not -CH₂-CO- or -(CH₂)₂-. In another aspect when -U-V- is -CH₂-CO- or -(CH₂)₂-, R⁵ is not indolyl, quinolinyl, 1,3-dihydro-2-oxo-benzimidazolyl or benzothienyl.

In another aspect one ofU and V are selected from CO, SO₂ and CH₂ and the other is CH₂.

Preferably Z⁵ is CH or N, Z³ is CH or CF and Z¹, Z² and Z⁴ are each CH, or Z¹ is N, Z³ is CH or CF and Z², Z⁴ and Z⁵ are each CH

When R¹ or R^{1a} is substituted alkoxy it is preferably (C₂₋₆)alkoxy substitituted by optionally N-substituted amino, guanidino or amidino, or (C₁₋₆)alkoxy substituted by piperidyl. Suitable examples of R¹ alkoxy include methoxy, n-propyloxy, i-butyloxy, aminoethyloxy, aminopropyloxy, aminobutyloxy, aminopentyloxy, guanidinopropyloxy, piperidin-4-ylmethyloxy, phthalimido pentyloxy or 2-aminocarbonylprop-2-oxy.

Preferably R¹ is methoxy, amino(C₃₋₅)alkyloxy, guanidino(C₃₋₅)alkyloxy, piperidyl(C₃₋₅)alkyloxy, nitro or fluoro; more preferably methoxy, amino(C₃₋₅)alkyloxy or guanidino(C₃₋₅)alkyloxy. Preferably R^{1a} is H or F. Most preferably R¹ is methoxy and R^{1a} is H or when Z³ is CR^{1a} it may be C-F.

When Z⁵ is CR^{1a}, R^{1a} is preferably hydrogen, cyano, hydroxymethyl or carboxy, most preferably hydrogen.

Preferably n is 0.

Preferred examples of R³ include hydrogen; (C₁₋₄) alkyl; ethenyl; optionally substituted 1-hydroxy-(C₁₋₄) alkyl; optionally substituted aminocarbonyl; carboxy(C₁₋₄) alkyl; optionally substituted aminocarbonyl(C₁₋₄)alkyl; cyano(C₁₋₄)alkyl; optionally substituted 2-oxo-oxazolidinyl and optionally substituted 2-oxo-oxazolidinyl(C₁₋₄alkyl). More preferred R³ groups are hydrogen; CONH₂; 1-hydroxyalkyl e.g. CH₂OH, CH(OH)CH₂CN; CH₂CO₂H; CH₂CONH₂; -CONHCH₂CONH₂; 1,2-dihydroxyalkyl e.g. CH(OH)CH₂OH; CH₂CN; 2-oxo-oxazolidin-5-yl and 2-oxo-oxazolidin-5-yl(C₁₋₄) alkyl). Most preferably R³ is hydrogen.

When R³ and R⁶, R⁷, R⁸ or R⁹ together form a cyclic ester or amide linkage, it is preferred that the resulting ring is 5-7 membered. It is further preferred that the group A or B which does not form the ester or amide linkage is CH₂.

When A is CH(OH) the R-stereochemistry is preferred.

Preferably A is NH, NCH₃, CH₂, CHOH, CH(NH₂), C(Me)(OH) or CH(Me).

Preferably B is CH₂ or CO.

Preferably n=0.

Most preferably:
n is 0 and either A is CHOH and B is CH₂ or A is NH and B is CO.

Preferably R¹¹ is hydrogen or (C₁₋₄)alkyl e.g. methyl, more preferably hydrogen.

Examples of-U-V- include -(CH₂)₂-, -CH₂CH(OH) and -CH₂CO-. The group -U-V- is preferably -(CH₂)₂-.

Preferably R⁵ is an aromatic heterocyclic ring (A) having 8-11 ring atoms including 2-4 heteroatoms of which at least one is N or NR¹³.

Alternatively and preferably the heterocyclic ring (A) has ring (a) aromatic and ring (b) non-aromatic and Y² has 3-5 atoms including NR¹³, O or S bonded to X⁵ and NHCO bonded via N to X³, or O or NH bonded to X³. Examples of rings (A) include optionally substituted:

### (a) and (b) aromatic

1H-pyrrolo[2,3-b]-pyridin-2-yl, 1H-pyrrolo[3,2-b]-pyridin-2-yl, 3H-imidazo[4,5-b]-pyrid-2-yl, 3H-quinazolin-4-one-2-yl, benzimidazol-2-yl, benzo[1,2,3]-thiadiazol-5-yl, benzo[1,2,5]-oxadiazol-5-yl, benzofur-2-yl, benzothiazol-2-yl, benzo[b]thiophen-2-yl, benzoxazol-2-yl, chromen-4-one-3-yl, imidazo[1,2-a]pyridin-2-yl, imidazo-[1,2-a]-pyrimidin-2-yl, indol-2-yl, indol-6-yl, isoquinolin-3-yl, [1,8]-naphthyridine-3-yl, oxazolo[4,5-b]-pyridin-2-yl, quinolin-2-yl, quinolin-3-yl, quinoxalin-2-yl, indan-2-yl and naphthalen-2-yl, 1,3-dioxo-isoindol-2yl, benzimidazol-2-yl, benzothiophen-2-yl,1H-benzotrizol-5-yl, 1H-indol-5-yl, 3H-benzooxazol-2-one-6-yl, 3H-benzothiazol-2-one-5-yl, 3H-quinazolin-4-one-2-yl, 3H-quinazolin-4-one-6-yl, 4-oxo-4H-pyrido[1,2-a]pyrimidin-3-yl, benzo[1,2,3]thiadiazol-6-yl, benzo[1,2,5]thiadiazol-5-yl, benzo[1,4]oxazin-2-one-3-yl, benzothiazol-5-yl, benzothiazol-6-yl, cinnolin-3-yl, imidazo[1,2-a]pyridazin-2-yl, imidazo[1,2-b]pyridazin-2-yl, pyrazolo[1,5-a]pyrazin-2-yl, pyrazolo[1,5-a]pyridin-2-yl, pyrazolo[1,5-a]pyrimidin-6-yl, pyrazolo[5,1-c][1,2,4]triazin 3-yl, pyrido[1,2-a]pyrimdin-4-one-2-yl, pyrido[1,2-a]pyrimidin-4-one-3-yl, quinazolin-2-yl, quinoxalin-6-yl, thiazolo[3,2-a]pyrimidin-5-one-7-yl, thiazolo[5,4-b]pyridin-2-yl, thieno[3,2-b]pyridin-6-yl, [1,2,3]thiadiazolo[5,4-b]pyridin-6-yl, isoindole-1,3-dione-2-yl,

### (a) is non aromatic

(2S)-2,3-dihydro-1H-indol-2-yl, (2S)-2,3-dihydio-benzo[t,4]dioxine-2-yt, 3-(R,S)-3,4-dihydro-2H-benzo[1,4]thiazin-3-yl, 3-(R)-(2,3-dihydro-[1,4]dioxino[2,3-b]pyridin-3-yl, 3-(S)-(2,3-dihydro-[1,4]dioxino[2,3-b]pyridin-3-yl, 2,3-dihydro-benzo[1,4]dioxan-2-yl,

### (b) is non aromatic

1,1,3-trioxo-1,2,3,4-tetrahydro-1 1⁶-benzo[1,4] thiazin-6-yl, benzo[1,3]dioxol-5-yl, 4H-benao[1,4]oxaain-3-one-6-yl, 2,3-dihydro-benzo[1,4]dioxin-6-yl, 2-oxo-2,3-dihydro-benzooxazol-6-yl, 3H-benzooxazol-2-one-6-yl, 3H-benzooxazole-2-thione-6-yl, 3H-benzothiazol-2-one-6-yl, 4H-benzo[1,4]oxazin-3-one-6-yl (3-oxo-3,4-dihydro-2H-benzo[1,4]oxazin-6-yl), 4H-benzo[1,4]thiazin-3-one-6-yl (3-oxo-3,4-dihydro-2H-beazo[1,4]thiazin-6 yl), 4H-benzo[1,4]oxazin-3-one-7-yl, 4-Oxo-2,3,4,5-tetrahydro-benzo[b][1,4]thiazepine-7-yl, 5-oxo-2,3-dihydro-5H-thiazolo[3,2-a]pyrimidin-6-yl, benzo[1,3]dioxol-5-yl, 2-oxo-2,3-dihydro-1H-pyrido[2,3-b]thiazin-7-yl, 2,3-dihydro-[1,4]dioxino[2,3-b]pyridin-6-yl, 2,3-dihydro-[1,4]dioxino[2,3-c]pyridin-7-yl, 2,3-dihydro-[1,4]dioxino[2,3-b]pyridin-7-yl, 3,4-dihydro-2H-benzo[1,4]oxazin-6-yl, 3,4-dihydro-2H-benzo[1,4]thiazin-6-yl, 3-oxo-3,4-dihydro-2H-pyrido[3,2-b][1,4]oxazin-6-yl, 2-oxo-2,3-dihydro-1H-pyrido[3,4-b]thiazin-7-yl, 3-oxo-3,4-dihydro-2H-pyrido[3,2-b][1,4]thiazin-6-yl, 2,3-dihydro-1H-pyrido[2,3-b][1,4]thiazin-7-yl, 3,4-dihydro-2H-pyrido[3,2-b][1,4]thiazin-6-yl, 5,6,7,8-tetrahydro-[1,8]naphthyridin-2-yl.

R¹³ is preferably H if in ring (a) or in addition (C₁₋₄)alkyl such as methyl or isopropyl when in ring (b). More preferably, in ring (b) R¹³ is H when NR¹³ is bonded to X³ and (C₁₋₄)alkyl when NR¹³ is bonded to X⁵.

R¹⁴ and R¹⁵ are preferably independently selected from hydrogen, halo, hydroxy, (C₁₋₄)alkoxy, trifluoromethoxy, nitro, cyano, aryl(C₁₋₄)alkoxy and (C₁₋₄)alkylsulphonyl.

More preferably R¹⁵ is hydrogen and each R¹⁴ is selected from hydrogen, chloro, fluoro, hydroxy, methoxy, trifluoromethoxy, benzyloxy, nitro, cyano and methylsulphonyl. Most preferably R¹⁴ is selected from hydrogen, hydroxy, fluorine or nitro. Preferably 0-3 groups R¹⁴ are substituents other than hydrogen.

More preferred groups R⁵ include:
4,6-difluoro-indol-2-yl,
1H-pyrrolo[2,3-b]-pyridin-2-yl,
1H-pyrrolo[3,2-b]-pyridin-2-yl,
8-hydroxy-quinolin-2-yl,
quinoxalin-2-yl,
benzimidazol-2-yl,
benzo[1,2,3]-thiadiazol-5-yl,
4- or 5-fluorobenzimidazol-2-yl,
benzothiophen-2-yl,
4,6-difluoro-1H-benzimidazol-2-yl,
2,2-difluoro-benzo[1,3]dioxol-5-yl,
2,3-Dihydro-benzo[1,4]dioxin-6-yl,
4H-benzo[1,4] oxazin-3-one-6-yl,
4H-benzo[1,4] thiazin-3-one-6-yl,
6-chloro-benzo[1,3]dioxol-5-yl,
7-fluoro-4H-benzo[1,4] oxazin-3-one-6-yl, and
benzo[1,3]dioxol-5-yl.

Most preferred groups R⁵ are:
4-fluorobenzimidazol-2-yl,
2,3-Dihydro-benzo[1,4]dioxin-6-yl,
4H-benzo[1,4] thiazin-3-one-6-yl.

When used herein, the term "alkyl" includes groups having straight and branched chains, for instance, methyl, ethyl, n-propyl, iso-propyl, n-butyl, iso-butyl, sec-butyl, t-butyl, pentyl and hexyl. The term 'alkenyl' should be interpreted accordingly.

Halo or halogen includes fluoro, chloro, bromo and iodo.

Haloalkyl moieties include 1-3 halogen atoms.

Each heterocyclic ring preferably has 5 or 6 ring atoms. A fused heterocyclic ring system may include carbocyclic rings and need include only one heterocyclic ring. Compounds within the invention containing a heterocyclyl group may occur in two or more tautometric forms depending on the nature of the heterocyclyl group; all such tautomeric forms are included within the scope of the invention.

'Aryl' groups are preferably optionally substituted with up to three groups as defined.

Some of the compounds of this invention may be crystallised or recrystallised from solvents such as organic solvents. In such cases solvates may be formed. This invention includes within its scope stoichiometric solvates including hydrates as well as compounds containing variable amounts of water that may be produced by processes such as lyophilisation.

Since the compounds of formula (I) are intended for use in pharmaceutical compositions it will readily be understood that they are each provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions; these less pure preparations of the compounds should contain at least 1%, more suitably at least 5% and preferably from 10 to 59% of a compound of the formula (I) or pharmaceutically acceptable salt and/or N-oxide thereof.

Pharmaceutically acceptable derivatives of the above-mentioned compounds of formula (I) include the free base form or their acid addition or quaternary ammonium salts, for example their salts with mineral acids e.g. hydrochloric, hydrobromic, sulphuric nitric or phosphoric acids, or organic acids, e.g. acetic, fumaric, succinic, maleic, citric, benzoic, p-toluenesulphonic, methanesulphonic, naphthalenesulphonic acid or tartaric acids. Compounds of formula (I) may also be prepared as the N-oxide. The invention extends to all such derivatives.

Certain of the above-mentioned compounds of formula (I) may exist in the form of optical isomers, e.g. diastereoisomers and mixtures of isomers in all ratios, e.g. racemic mixtures. The invention includes all such forms, in particular the pure isomeric forms. For examples the invention includes compound in which an A-B group CH(OH)-CH₂ is in either isomeric configuration the R-isomer is preferred. The different isomeric forms may be separated or resolved one from the other by conventional methods, or any given isomer may be obtained by conventional synthetic methods or by stereospecific or asymmetric syntheses..

In a further aspect of the invention there is provided a process for preparing compounds of formula (I), or a pharmaceutically acceptable salt and/or N-oxide thereof, which process comprises reacting a compound of formula (IV) with a compound of formula (V): 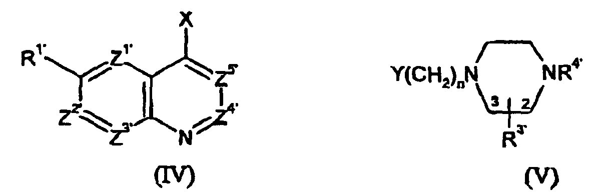 wherein n is as defined in formula (I); Z^{1'}, Z^{2'}, Z^{3'}, Z^{4'}, Z^{5'}, R^{1'}, R^{3'} and R^{4'} are Z¹, Z², Z³, Z⁴, Z⁵, R¹, R³ and R⁴ as defined in formula (I) or groups convertible thereto; and X and Y may be the following combinations:
(i) X is A'-COW, Y is H and n is 0;
(ii) X is CR⁶=CR⁸R⁹, Y is H and n is 0;
(iii) X is oxirane, Y is H and n is 0;
(iv) X is N=C=O and Y is H and n is 0;
(v) one of X and Y is CO₂R^{y} and the other is CH₂CO₂R^{x};
(vi) X is CHR⁶R⁷ and Y-is C(=O)R⁸;
(vii) X is CR⁷=PR^{z}₃ and Y is C(=O)R⁹ and n=1;
(viii) X is C(=O)R⁷ and Y is CR⁹=PR^{z}₃ and n=1;
(ix) Y is COW and X is NHR^{11'} or NR11'COW and n=0 or 1 or when n=1 X is COW and Y is NHR^{11'} or NR11'COW;
(x) X is NHR^{11'} and Y is C(=O)R⁸ and n=1;
(xi) X is NHR^{11'} and Y is CR⁸R⁹W and n=1;
(xii) X is NR^{11'}COCH₂W or NR^{11'}SO₂CH₂W and Y is H and n=0;
(xiii) X is CR⁶R⁷SO₂W and Y is H and n=0;
(xiv) X is W or OH and Y is CH₂OH and n is 1;
(xv) X is NHR^{11'} and Y is SO₂W or X is NR^{11'}SO₂W and Y is H, and n is 0;
in which W is a leaving group, e.g. halo or imidazolyl; R^{x} and R^{y} are (C₁₋₆)alkyl; R^{z} is aryl or (C₁₋₆)alkyl; A' and NR^{11'} are A and NR¹¹ as defined in formula (I), or groups convertible thereto; and oxirane is: 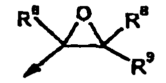 wherein R⁶, R⁸ and R⁹ are as defined in formula (I);
and thereafter optionally or as necessary converting A', Z^{1'}, Z^{2'}, Z^{3'}, Z^{4'}, Z^{5'}, R^{1'}, R^{3'}, R^{4'} and NR^{11'} to A, Z¹, Z², Z³, Z⁴, Z⁵, R¹, R³, R⁴ and NR¹¹; converting A-B to other A-B, interconverting R¹, R³ and/or R⁴, and/or forming a pharmaceutically acceptable salt and/or N-oxide thereof.

Process variant (i) initially produces compounds of formula (I) wherein A-B is A'-CO.

Process variant (ii) initially produces compounds of formula (I) wherein A-B is CHR⁶-CR⁸R⁹.

Process variant (iii) initially produces compounds of formula (I) wherein A-B is CR⁶(OH)-CR⁸R⁹.

Process variant (iv) initially produces compounds of formula (I) where A-B is NH-CO.

Process variant (v) initially produces compounds of formula (I) wherein A-B is CO-CH₂ or CH₂-CO.

Process variant (vi) initially produces compounds of formula (I) wherein A-B is CR⁶R⁷-CR⁸OH.

Process variant (vii) and (viii) initially produce compounds of formula (I) wherein A-B is CR⁷=CR⁹.

Process variant (ix) initially produces compounds of formula (I) where A-B is CO-NR¹¹ or NR¹¹-CO.

Process variant (x) initially produces compounds of formula (I) wherein A-B is NR¹¹-CHR⁸.

Process variant (xi) initially produces compounds of formula (I) wherein A-B is NR^{11'}-CR₈R₉.

Process variant (xii) initially produces compounds of formula (I) where A-B is NR^{11'}-CO or NR^{11'}-SO₂ and n=1.

Process variant (xiii) initially produces compounds of formula (I) where A-B is CR⁶R⁷-SO₂.

Process variant (xiv) initially produces compounds of formula (I) wherein A-B is O-CH₂.

Process variant (xv) initially produces compounds where AB is NR¹¹SO₂.

In process variants (i) and (ix) the reaction is a standard amide or urea formation reaction involving e.g.:
1. Activation of a carboxylic acid (e.g. to an acid chloride, mixed anhydride, active ester, O-acyl-isourea or other species), and treatment with an amine (Ogliaruso, M.A.; Wolfe, J.F. in *The Chemistry of Functional Groups (Ed. Patai, S.) Suppl. B: The Chemistry of Acid Derivatives, Pt. 1* (John Wiley and Sons, 1979), pp 442-8; Beckwith, A.L.J. in *The Chemistry of Functional Groups (Ed Patai, S.) Suppl. B: The Chemistry of Amides (Ed. Zabricky, J.)* (John Wiley and Sons, 1970), p 73 ff. The acid and amine are preferably reacted in the presence of an activating agent such as 1-(dimethylaminopropyl)-3-ethylcarbodiimide hydrochloride (EDC) or 1-hydroxybenzotriazole (HOBT); or
2. The specific methods of:
   a. *in situ* conversion of an acid into the amine component by a modified Curtius reaction procedure (Shioiri, T., Murata, M., Hamada, Y., *Chem. Pharm. Bull.* 1987, 35, 2698)
   b. *in situ* conversion of the acid component into the acid chloride under neutral conditions (Villeneuve, G. B.; Chan, T. H., *Tetrahedron. Lett.* 1997, 38, 6489).

A' may be, for example, protected hydroxymethylene.

The process variant (ii) is a standard addition reaction using methods well known to those skilled in the art. The process is preferably carried out in a polar organic solvent e.g. acetonitrile in the presence of an organic base e.g. triethylamine.

In process variant (iii) the coupling may be effected in acetonitrile at room temperature in the presence of one equivalent of lithium perchlorate as catalyst (general method of J.E. Chateauneuf *et al, J. Org. Chem.,* 56, 5939-5942, 1991) or more preferably with ytterbium triflate in dichloromethane. In some cases an elevated temperature such as 40 - 70 °C may be beneficial. Alternatively, the piperazine may be treated with a base, such as one equivalent of butyl lithium, and the resulting salt reacted with the oxirane in an inert solvent such as tetrahydrofuran, preferably at an elevated temperature such as 80°C. Use of a chiral epoxide will afford single diastereomers. Alternatively, mixtures of diastereomers may be separated by preparative HPLC or by conventional resolution through crystallisation of salts formed from chiral acids.

The process variant (iv) is a standard urea formation reaction from the reaction of an isocyanate with an amine and is conducted by methods well known to those skilled in the art (for example see March, J; *Advanced Organic Chemistry, Edition 3* (John Wiley and Sons, 1985), p802-3). The process is preferably carried out in a polar solvent such as N,N-dimethylformamide.

In process variant (v) the process is two step: firstly a condensation using a base, preferably sodium hydride or alkoxide, sodamide, alkyl lithium or lithium dialkylamide, preferably in an aprotic solvent e.g. ether, THF or benzene; secondly, hydrolysis using an inorganic acid, preferably HCl in aqueous organic solvent at 0-100°C. Analogous routes are described in DE330945, EP31753, EP53964 and H. Sargent, J. Am. Chem. Soc. 68, 2688-2692 (1946). Similar Claisen methodology is described in Soszko et. al., Pr.Kom.Mat. Przyr.Poznan.Tow.Przyj.Nauk., (1962),10,15.

In process variant (vi) the reaction is carried out in the presence of a base, preferably organometallic or metal hydride e.g. NaH, lithium diisopropylamide or NaOEt, preferably in an aprotic solvent, preferably THF, ether or benzene at -78 to 25°C (analogous process in Gutswiller et al. (1978) J. Am. Chem. Soc. 100, 576).

In process variants (vii) and (viii) if a base is used it is preferably NaH, KH, an alkyl lithium e.g. BuLi, a metal alkoxide e.g. NaOEt, sodamide or lithium dialkylamide e.g.di- isopropylamide. An analogous method is described in US 3989691 and M.Gates et. al. (1970) J. Amer.Chem.Soc., 92, 205, as well as Taylor et al. (1972) JACS 94, 6218.

In process variant (x) where X or Y is CHO the reaction is a standard reductive alkylation using, e.g., sodium borohydride or sodium triacetoxyborohydride (Gribble, G. W. in *Encyclopedia of Reagents for Organic Synthesis (Ed. Paquette, L. A.)* (John Wiley and Sons, 1995), p 4649).

The process variant (xi) is a standard alkylation reaction well known to those skilled in the art, for example where an alcohol or amine is treated with an alkyl halide in the presence of a base (for example see March, J; *Advanced Organic Chemistry, Edition 3* (John Wiley and Sons, 1985), p364-366 and p342-343). The process is preferably carried out in a polar solvent such as N,N-dimethylformamide.

In process variant (xii) the reaction is an alkylation, examples of which are described in J. Med. chem. (1979) 22(10) 1171-6. The compound of formula (IV) may be prepared from the corresponding compound where X is NHR^{11'} by acylation with an appropriate derivative of the acid WCH₂COOH such as the acid chloride or sulphonation with an appropriate derivative of the sulphonic acid WCH₂SO₃H such as the sulphonyl chloride.

In process variant (xiii) the reaction is a standard sulphonamide formation reaction well known to those skilled in the art. This may be e.g. the reaction of a sulphonyl halide with an amine.

In process variant (xiv) where X is W such as halogen, methanesulphonyloxy or trifluoromethanesulphonyloxy, the hydroxy group in Y is preferably converted to an OM group where M is an alkali metal by treatment of an alcohol with a base. The base is preferably inorganic such as NaH, lithium diisopropylamide or sodium. Where X is OH, the hydroxy group in Y is activated under Mitsunobu conditions (Fletcher et.al. J Chem Soc. (1995), 623). Alternatively the X=O and Y=CH₂OH groups can be reacted directly by activation with dichlorocarbodiimide (DCC) (Chem. Berichte 1962, 95, 2997 or Angewante Chemie 1963 75, 377).

In process variant (xv) the reaction is conducted in the presence of an organic base such as triethylamine or pyridine such as described by Fuhrman et.al., J. Amer. Chem. Soc.; **67**, 1245, 1945. The X=NR^{11'}SO₂W or Y=SO₂W intermediates can be formed from the requisite amine e.g. by reaction with SO₂Cl₂ analogously to the procedure described by the same authors Fuhrman et.al., J. Amer. Chem. Soc.; **67**, 1245, 1945.

Reduction of a carbonyl group A or B to CHOH can be readily accomplished using reducing agents well known to those skilled in the art, e.g. sodium borohydride in aqueous ethanol or lithium aluminium hydride in ethereal solution. This is analogous to methods described in EP53964, US384556 and J. Gutzwiller *et al, J. Amer. Chem. Soc.,* 1978, 100, 576.

The carbonyl group A or B may be reduced to CH₂ by treatment with a reducing agent such as hydrazine in ethylene glycol, at e.g. 130-160°C, in the presence of potassium hydroxide.

Reaction of a carbonyl group A or B with an organometallic reagent yields a group where R⁶ or R⁸ is OH and R⁷ or R⁹ is alkyl.

A hydroxy group on A or B may be oxidised to a carbonyl group by oxidants well known to those skilled in the art, for example, manganese dioxide, pyridinium chlorochromate or pyridinium dichromate.

A hydroxyalkyl A-B group CHR⁶CR⁸OH or CR⁶(OH)CHR⁸ may be dehydrated to give the group CR⁶=CR⁸ by treatment with an acid anhydride such as acetic anhydride.

Methods for conversion of CR⁶=CR⁸ by reduction to CHR⁶CHR⁸ are well known to those skilled in the art, for example using hydrogenation over palladium on carbon as catalyst. Methods for conversion of CR⁶=CR⁸ to give the A-B group CR⁶(OH)CHR⁸ or CHR⁶CR⁸OH are well known to those skilled in the art for example by epoxidation and subsequent reduction by metal hydrides, hydration, hydroboration or oxymercuration.

An amide carbonyl group may be reduced to the corresponding amine using a reducing agent such as lithium aluminium hydride.

A hydroxy group in A or B may be converted to azido by activation and displacement e.g. under Mitsunobu conditions using hydrazoic acid or by treatment with diphenylphosphorylazide and base, and the azido group in turn may be reduced to amino by hydrogenation.

Examples of groups Z^{1'}, Z^{2'}, Z^{3'}, Z^{4'}, Z^{5'}, are CR^{1a'} where R^{1a'} is a group convertible to R^{1a}, Z^{1'}, Z^{2'}, Z^{3'}, Z^{4'} and Z^{5'} are preferably Z¹, Z², Z³, Z⁴ and Z⁵.

R^{1a'} and R^{1'} are preferably R^{1a} and R¹. R^{1'} is preferably methoxy. R^{3'} is R³ or more preferably hydrogen, vinyl, alkoxycarbonyl or carboxy. R^{4'} is R⁴ or more preferably H or an N-protecting group such as t-butoxycarbonyl, benzyloxycarbonyl or 9-fluorenylmethyloxycarbonyl.

Conversions of R^{1a'}, R^{1'}, R^{3'} and R^{4'} and interconversions of R^{1a}, R¹, R³ and R⁴ are conventional. In compounds which contain an optionally substituted hydroxy group, suitable conventional hydroxy protecting groups which may be removed without disrupting the remainder of the molecule include acyl and alkylsilyl groups.

For example R^{1'} methoxy is convertible to R^{1'} hydroxy by treatment with lithium and diphenylphosphine (general method described in Ireland et. al. (1973) J.Amer.Chem.Soc.,7829) or HBr. Alkylation of the hydroxy group with a suitable alkyl derivative bearing a leaving group such as halide and a protected amino, piperidyl, amidino or guanidino group or group convertible thereto, yields, after conversion/deprotection, R¹ alkoxy substituted by optionally N-substituted amino, piperidyl, guanidino or amidino.

R³ alkenyl is convertible to hydroxyalkyl by hydroboration using a suitable reagent such as 9-borabicyclo[3.3.1]nonane, epoxidation and reduction or oxymercuration.

R³ 1,2-dihydroxyalkyl can be prepared from R^{3'} alkenyl using osmium tetroxide or other reagents well known to those skilled in the art (see Advanced Organic Chemistry *(Ed. March, J.)* (John Wiley and Sons, 1985), p 732-737 and refs. cited therein) or epoxidation followed by hydrolysis (see Advanced Organic Chemistry *(Ed. March, J.)* (John Wiley and Sons, 1985), p 332,333 and refs. cited therein).

R³ vinyl can be chain extended by standard homologation e.g by conversion to hydroxyethyl followed by oxidation to the aldehyde which is then subjected to a Wittig reaction.

Opening an epoxide R^{3'} group with cyanide anion yields a CH(OH)-CH₂CN group.

Opening an epoxide-containing R^{3'} group with azide anion yields an azide derivative which can be reduced to the amine. Conversion of the amine to a carbamate is followed by ring closure with base to give the 2-oxo-oxazolidinyl containing R³ group.

Substituents on R³ alkyl or alkenyl may be interconverted by conventional methods, for example hydroxy may be derivatised by esterification, acylation or etherification. Hydroxy groups may be converted to halogen, thiol, alkylthio, azido, alkylcarbonyl, amino, aminocarbonyl, oxo, alkylsulphonyl, alkenylsulphonyl or aminosulphonyl by conversion to a leaving group and substitution by the required group, hydrolysis or oxidation as appropriate or reaction with an activated acid, isocyanate or alkoxyisocyanate. Primary and secondary hydroxy groups can be oxidised to an aldehyde or ketone respectively and alkyated with a suitable agent such as an organometallic reagent to give a secondary or tertiary alcohol as appropriate. A carboxylate group may be converted to an hydroxymethyl group by reduction of an ester of this acid with a suitable reducing agent such as lithium aluminium hydride.

Substituted 2-oxo-oxazolidinyl containing R³ groups may be prepared from the corresponding aldehyde by conventional reaction with a glycine anion equivalent, followed by cyclisation of the resulting amino alcohol (M Grauert et al, Ann Chem (1985) 1817, Rozenberg et al, Angew Chem Int Ed Engl (1994) 33(1) 91). The resulting 2-oxo-oxazolidinyl group contains a carboxy group which can be converted to other R¹⁰ groups by standard procedures.

Carboxy groups within R³ may be prepared by Jones' oxidation of the corresponding alcohols CH₂OH using chromium acid and sulphuric acid in water/methanol (E.R.H. Jones et al, J.C.S. 1946,39). Other oxidising agents may be used for this transformation such as sodium periodate catalysed by ruthenium trichloride (G.F.Tutwiler *et al,* J.Med.Chem., 1987, *30*(6), 1094), chromium trioxide-pyridine (G. Just *et al,* Synth. Commun. 1979, 9(7), 613), potassium permanganate (D.E.Reedich *et al,* J. Org. Chem.,1985,*50*(19),3535, and pyridinium chlorochromate (D. *Askin et al,* Tetrahedron Letters, 1988, 29(3), 277.

The carboxy group may alternatively be formed in a two stage process, with an initial oxidation of the alcohol to the corresponding aldehyde using for instance dimethyl sulphoxide activated with oxalyl chloride (N.Cohen *et al,* J. Am. Chem. Soc., 1983, 105, 3661) or dicyclohexylcarbodiimide (R.M.Wengler, Angew. Chim. Int. Ed. Eng., 1985, 24(2), 77), or oxidation with tetrapropylammonium perruthenate (Ley *et al,* J. Chem.Soc. Chem Commun.,1987, 1625). The aldehyde may then be separately oxidised to the corresponding acid using oxidising agents such as silver (II) oxide (R.Grigg *et al,* J. Chem. Soc. Perkin 1, 1983, 1929), potassium permanganate (A.Zurcher, Helv. Chim. Acta., 1987, 70 (7), 1937), sodium periodate catalysed by ruthenium trichloride (T.Sakata *et al,* Bull. Chem. Soc. Jpn., 1988, 61(6), 2025), pyridinium chlorochromate (R.S.Reddy *et al,* Synth. Commun., 1988, 18(51), 545) or chromium trioxide (R.M.Coates *et al,* J. Am. Chem. Soc.,1982, 104, 2198).

An R³ CO₂H group may also be prepared from oxidative cleavage of the corresponding diol, CH(OH)CH₂OH, using sodium periodate catalysed by ruthenium trichloride with an acetontrile-carbontetrachloride-water solvent system (V.S.Martin *et al,* Tetrahedron Letters, 1988, 29(22), 2701).

R³ groups containing a cyano or carboxy group may also be prepared by conversion of an alcohol to a suitable leaving group such as the corresponding tosylate by reaction with para-toluenesulphonyl chloride (M.R.Bell, J. Med. Chem., 1970, 13, 389), or the iodide using triphenylphosphine, iodine, and imidazole (G. Lange, Synth. Commun., 1990, 20, 1473). The second stage is the displacement of the leaving group with cyanide anion (LA.Paquette et al, J. Org. Chem.,1979, 44 (25), 4603; P.A.Grieco et al, J. Org. Chem.,1988, 53 (16), 3658). Finally acidic hydrolysis of the nitrile group gives the desired acids (H.Rosemeyer et al, Heterocycles, 1985, 23 (10), 2669). The hydrolysis may also be carried out with base e.g. potassium hydroxide (H.Rapoport, J. Org. Chem.,1958, 23, 248) or enzymatically (T. Beard et al, Tetrahedron Asymmetry, 1993, 4 (6), 1085).

Other functional groups in R³ may be obtained by conventional conversions of carboxy or cyano groups.

Tetrazoles are conveniently prepared by reaction of sodium azide with the cyano group (e.g. F. Thomas et al, Bioorg. Med. Chem. Lett., 1996, 6 (6), 631; K.Kubo et al, J. Med. Chem., 1993, 36,2182) or by reaction of azidotri-n-butyl stannane with the cyano group followed by acidic hydrolysis (P.L.Ornstein, J. Org. Chem., 1994, 59, 7682 and J. Med. Chem, 1996, 39 (11), 2219).

The 3-hydroxy-3-cyclobutene-1,2-dion-4-yl group (e.g. R.M.Soll, Bioorg. Med. Chem. Lett., 1993, 3 (4), 757 and W. A. Kinney, J. Med. Chem., 1992, 35 (25), 4720) can be prepared by the following sequence:- (1) a compound where R3 is (CH₂)ₙCHO (n = 0,1,2) is treated with triethylamine, carbon tetrabromide triphenylphosphine to give initially (CH₂)ₙCH=CBr₂; (2) dehydrobromination of this intermediate to give the corresponding bromoethyne derivative (CH₂)ₙC≡CBr (for this 2 stage sequence see D. Grandjean et al, Tetrahedron Letters, 1994, 35 (21), 3529); (3) palladium-catalysed coupling of the bromoethyne with 4-(1-methylethoxy)-3-(tri-n-butylstannyl)cyclobut-3-ene-1,2-dione (Liebeskind et al, J. Org. Chem., 1990, 55, 5359); (4) reduction of the ethyne moity to -CH2CH2- under standard conditions of hydrogen and palladium on charcol catalysis(see Howard et al, Tetrahedron, 1980, *36*, 171); and finally (4) acidic hydrolysis of the methylethoxyester to generate the corresponding 3-hydroxy-3-cyclobutene-1,2-dione group R.M.Soll, Bioorg. Med. Chem. Lett., 1993, 3 (4), 757).

The tetrazol-5-ylaminocarbonyl group may be prepared from the corresponding carboxylic acid and 2-aminotetrazole by dehydration with standard peptide coupling agents such as 1,1'-carbonyldiimidazole (P. L. Ornstein et al, J. Med Chem, 1996, *39* (11), 2232).

The alkyl- and alkenyl-sulphonylcarboxamides are similarly prepared from the corresponding carboxylic acid and the alkyl- or alkenyl-sulphonamide by dehydration with standard peptide coupling agents such as 1,1'-carbonyldiimidazole (P. L. Ornstein et al, J.Med.Chem., 1996, 39 (11), 2232).

The hydroxamic acid groups are prepared from the corresponding acids by standard amide coupling reactions eg N. R. Patel et al, Tetrahedron, 1987, 43 (22), 5375

2,4-thiazolidinedione groups may prepared from the aldehydes by condensation with 2,4-thiazolidinedione and subsequent removal of the olefinic double bond by hydrogenation.

The preparation of 5-oxo-1,2,4-oxadiazoles from nitriles is decribed by Y.Kohara et al, Bioorg. Med. Chem. Lett., 1995, 5(17), 1903.

1,2,4-triazol-5-yl groups may be prepared from the corresponding nitrile by reaction with an alcohol under acid conditions followed by reaction with hydrazine and then an R¹⁰-substituted activated carboxylic acid (see JB Polya in 'Comprehensive Heterocyclic Chemistry' Edition 1 p762, Ed AR Katritzky and CW Rees, Pergamon Press, Oxford 1984 and J.J. Ares et al, J. Heterocyclic Chem., 1991, 28(5), 1197).

NH is converted to NR⁴ by conventional means such as amide or sulphonamide formation with an acyl derivative R⁵V'COW or R⁵V'SO₂W, for compounds where U is CO or SO₂ or, where U is CH₂, by reaction with a vinyl derivative R⁵-CH=CH₂, for example by heating in an alcohol such as ethanol containing an acid such as acetic acid, alkylation with an alkyl halide R⁵-V'-CH₂-halide or alkyl derivative R⁵-V'-CH₂-W in the presence of base, acylation/reduction or reductive alkylation with an aldehyde R⁵-V'-CHO where V' is V or a group convertible thereto such as a dimethyl acetal or 1,3-dithiane. Such groups are deprotected by conventional means eg dimethyl acetal by hydrolysis using dilute acid in tetrahydrofuran, and 1,3-dithiane using HgCl₂ in aqueous acetonitrile (Marshall J. A. et al., J. Org. Chem. 34, 4188 (1969) or AgNO₂ and I₂ in tetrahydrofuran (Nishide K. et al., Heterocycles 44(1) 393 (1997).

Where V is a group CR¹⁷R¹⁸ this may be obtained by reduction of a V = CO group followed by derivatisation of the resulting R¹⁷ = OH group as necessary.

Where one of R³ and R⁶, R⁷, R⁸ or R⁹ contains a carboxy group and the other contains a hydroxy or amino group they may together form a cyclic ester or amide linkage. This linkage may form spontaneously during coupling of the compound of formula (IV) and the piperazine moiety or in the presence of standard peptide coupling agents.

It will be appreciated that under certain circumstances interconvertions may interfere, for example, hydroxy groups in A or B and the piperazine NH will require protection e.g. as a carboxy- or silyl-ester group for hydroxy and as an acyl derivative for piperidine nitrogen, during conversion of R^{1a'}, R^{1'}, R^{3'} or R^{4'}, or during the coupling of the compounds of formulae (IV) and (V).

Compounds of formulae (IV) and (V) are known compounds, (see for example Smith *et al, J. Amer. Chem. Soc.,* 1946, 68, 1301) or prepared analogously, see for example the references cited above for reaction variant (a).

Compounds of formula (IV) where X is CR⁶R⁷SO₂W may be prepared by a route analogous to that of Ahmed EI Hadri *et al, J. Heterocyclic Chem.,* 1993, 30(3), 631.
Thus compounds of formula (IV) where X is CH₂SO₂OH may be prepared by reacting the corresponding 4-methyl compound with N-bromosuccinimide, followed by treatment with sodium sulfite. The leaving group W may be converted to another leaving group W, e.g. a halogen group, by conventional methods.

The isocyanate of formula (IV) may be prepared conventionally from a 4-amino derivative such as 4-amino-quinoline, and phosgene, or phosgene equivalent (eg triphosgene) or it may be prepared more conveniently from a 4-carboxylic acid by a "one-pot" Curtius Reaction with diphenyl phosphoryl azide (DPPA) [see T. Shiori et al. *Chem. Pharm. Bull.* **35**, 2698-2704 (1987)].

The 4-amino derivatives are commercially available or may be prepared by conventional procedures from a corresponding 4-chloro derivative by treatment with ammonia (O.G. Backeberg et. al., J. Chem Soc., 381, 1942) or propylamine hydrochloride (R. Radinov et. al., Synthesis, 886, 1986).

4-Alkenyl compounds of formula (IV) may be prepared by conventional procedures from a corresponding 4-halogeno-derivative by e.g. a Heck synthesis as described in e.g. *Organic Reactions,* 1982, 27, 345.

4-Halogeno derivatives of compounds of formula (IV) are commercially available, or may be prepared by methods known to those skilled in the art. A 4-chloroquinoline is prepared from the corresponding quinolin-4-one by reaction with phosphorus oxychloride (POCl₃) or phosphorus pentachloride, PCl₅. A 4-chloroquinazoline is prepared from the corresponding quinazolin-4-one by reaction with phosphorus oxychloride (POCl₃) or phosphorus pentachloride, PCl₅. A quinazolinone and quinazolines may be prepared by standard routes as described by T.A. Williamson in *Heterocyclic Compounds,* 6, 324 (1957) Ed. R.C. Elderfield.

4-Carboxy derivatives of compounds of formula (IV) are commercially available or may be prepared by conventional procedures for preparation of carboxy heteroaromatics well known to those skilled in the art. For example, quinazolines may be prepared by standard routes as described by T.A. Williamson in *Heterocyclic Compounds,* 6, 324 (1957) Ed. R.C. Elderfield. These 4-carboxy derivatives may be activated by conventional means, e.g. by conversion to an acyl halide or anhydride.

Pyridazines may be prepared by routes analogous to those described in Comprehensive Heterocyclic Chemistry, Volume 3, Ed A.J. Boulton and A. McKillop and napthyridines may be prepared by routes analogous to those described in Comprehensive Heterocyclic Chemistry, Volume 2, Ed A.J. Boulton and A. McKillop.

A 4-oxirane derivative of compounds of formula (IV) is conveniently prepared from the 4-carboxylic acid by first conversion to the acid chloride with oxalyl chloride and then reaction with trimethylsilyldiazomethane to give the diazoketone derivative. Subsequent reaction with 5M hydrochloric acid gives the chloromethylketone. Reduction with sodium borohydride in aqueous methanol gives the chlorohydrin which undergoes ring closure to afford the epoxide on treatment with base, e.g. potassium hydroxide in ethanol-tetrahydrofuran.

Alternatively and preferably, 4-oxirane derivatives can be prepared from bromomethyl ketones which can be obtained from 4-hydroxy compounds by other routes well known to those skilled in he art. For example, hydroxy compounds can be converted to the corresponding 4-trifluoromethanesulphonates by reaction with trifluoromethanesulphonic anhydride under standard conditions (see K. Ritter, Synthesis, 1993, 735). Conversion into the corresponding butyloxyvinyl ethers can be achieved by a Heck reaction with butyl vinyl ether under palladium catalysis according to the procedure of W. Cabri *et al,* J. Org. Chem, 1992, **57** (5), 1481. (Alternatively, the same intermediates can be attained by Stille coupling of the trifluoromethanesulphonates or the analaogous chloro derivatives with (1-ethoxyvinyl)tributyl tin, T. R. Kelly, J. Org. Chem., 1996, **61**, 4623.) The alkyloxyvinyl ethers are then converted into the corresponding bromomethylketones by treatment with N-bromosuccinimide in aqueous tetrahydrofuran in a similar manner to the procedures of J. F. W. Keana, J. Org. Chem., 1983, 48, 3621 and T. R. Kelly, J. Org. Chem., 1996, 61, 4623.

The 4-hydroxyderivatives can be prepared from an aminoaromatic by reaction with methylpropiolate and subsequent cyclisation, analogous to the method described in N. E. Heindel et al, J. Het. Chem., 1969, 6, 77. For example, 5-amino-2-methoxy pyridine can be converted to 4-hydroxy-6-methoxy-[1,5]naphthyridine using this method.

If a chiral reducing agent such as (+) or (-)-B-chlorodiisopinocamphenylborane ['DIP-chloride'] is substituted for sodium borohydride, the prochiral chloromethylketone is converted into the chiral chlorohydrin with ee values generally 85-95% [see C. Bolm *et al, Chem. Ber.* 125, 1169-1190, (1992)]. Recrystallisation of the chiral epoxide gives material in the mother liquor with enhanced optical purity (typically ee 95%).

The (*R*)-epoxide, when reacted with a piperazine derivative gives ethanolamine compounds as single diastereomers with (R)-stereochemistry at the benzylic position.

Alternatively, the epoxide may be prepared from the 4-carboxaldehyde by a Wittig approach using trimethylsulfonium iodide [see G.A. Epling and K-Y Lin, *J. Het. Chem.,* 1987, 24, 853-857], or by epoxidation of a 4-vinyl derivative.

4-Hydroxy-1,5-naphthyridines can be prepared from 3-aminopyridine derivatives by reaction with diethyl ethoxymethylene malonate to produce the 4-hydroxy-3-carboxylic acid ester derivative with subsequent hydrolysis to the acid, followed by thermal decarboxylation in quinoline (as for example described for 4-Hydroxy-[1,5]naphthyridine-3-carboxylic acid, J. T. Adams *et al., J.Amer.Chem.Soc.,* 1946, 68, 1317). A 4-hydroxy-[1,5]naphthyridine can be converted to the 4-chloro derivative by heating in phosphorus oxychloride, or to the 4-methanesulphonyloxy or 4-trifluoromethanesulphonyloxy derivative by reaction with methanesulphonyl chloride or trifluoromethanesulphonic anhydride, respectively, in the presence of an organic base. A 4-amino 1,5-naphthyridine can be obtained from the 4-chloro, 4-methanesulphonyloxy or 4-trifluoromethanesulphonyloxy derivative by reaction with n-propylamine in pyridine.

Similarly, 6-methoxy-1,5-naphthyridine derivatives can be prepared from 3-amino-6-methoxypyridine.

1,5-Naphthyridines may be prepared by other methods well known to those skilled in the art (for examples see P.A. Lowe in "Comprehensive Heterocyclic Chemistry" Volume 2, p581-627, Ed A.R. Katritzky and C.W. Rees, Pergamon Press, Oxford, 1984).

The 4-hydroxy and 4-amino-cinnolines may be prepared following methods well known to those skilled in the art [see A.R. Osborn and K. Schofield, *J. Chem. Soc.* 2100 (1955)]. For example, a 2-aminoacetopheneone is diazotised with sodium nitrite and acid to produce the 4-hydroxycinnoline with conversion to chloro and amino derivatives as described for 1,5-naphthyridines.

The substituted piperazines of formula (V) are either commercially available or may be synthesised by hydrogenation of the corresponding pyrazines (eg von E. Felder et al. *Helv. Chim. Acta* **33,** 888-896 (1960)], or by diborane reduction of a suitable lactam [eg H.L. Larkins et al. *Tet. Lett.* **27,** 2721-2724 (1986)].
Chiral piperazines may be prepared from chiral 2-(S)- and 2-(R)-piperazinecarboxylic acid. Racemic piperazine-2-carboxylic acid (commercially available) may be resolved by crystallisation of the (S)- and (R)-dicamphor-10-sulfonic acid salts [following the general method of K. Stingl et al. *Tet. Asymmetry* **8**, 979-982 (1997)-described for preparation of 2-(S)-piperazinecarboxylic acid].

Piperazine-2-carboxylic acid may be differentially protected [following the procedure of C.F. Bigge et al. *Tet. Lett.* **30**, 5193-5196 (1989)] by first reacting with 2-(t-butoxycarbonyloxyimino)-2-phenylacetonitrile which selectively reacts on N-4, and then by reacting with benzylchloroformate which reacts on N-1. The 2-carboxylic acid is then methylated (conveniently with TMS-diazomethane). Hydrogenation (over Pd/C) then removes the carbobenzyloxy group, which may be alkylated or acylated with the required R⁴ group as described above for the conversion of R^{4'}=H to R⁴. Reaction with trifluoroacetic acid (optionally in dichloromethane) removes the N-4-butoxycarbonyloxy group to afford the required 4-H piperazine.

The chiral piperazine-2-carboxylic acids may be elaborated to various derivatives, for example, an Arndt-Eistert procedure (involving silver salt mediated rearrangement of a diazoketone) will give chiral 2-acetic acid derivatives (initially via the methyl ester). Reduction of the intermediate ester with standard reducing agents such as lithium aluminium hydride will produce a hydroxymethyl derivative.

Compounds of formula (V) where n=1 may be prepared from the compound where n=0 by homologation eg starting from a compound of formula (V) where Y=CO₂H.

R⁵-V'-CH₂-halides, acyl derivatives R⁵V'COW and R⁵V'SO₂W or aldehydes R⁵⁻V'-CHO, vinyl derivatives R⁵-CH=CH₂ and alkyl derivatives R⁵-V'-CH₂-W are commercially available or are prepared conventionally. The aldehydes may be prepared by partial reduction of the R⁵-V'-ester with lithium aluminium hydride or diisobutylaluminium hydride or more preferably by reduction to the alcohol, with lithium aluminium hydride or sodium borohydride, followed by oxidation to the aldehyde with manganese (II) dioxide (see *Reductions by the Alumino- and Borohydrides in Organic Synthesis,* 2nd ed., Wiley, N.Y., **1997;** *JOC,* 3197, **1984;** *Org. Synth. Coll.,* 102, **1990;** 136, **1998;** *JOC,* 4260, **1990;** *TL,* 995,**1988;** *JOC,* 1721, **1999;** *Liebigs Ann.*/*Recl.,* 2385, **1997**; *JOC,* 5486, **1987**). The aldehydes may also be prepared from carboxylic acids in two stages by conversion to a mixed anhydride for example by reaction with isobutyl chloroformate followed by reduction with sodium borohydride (R. J. Alabaster et al., Synthesis, 598, 1989) to give the alcohols and then oxidation with a standard oxidising agent such as pyridinium dichromate or by homologation of the R⁵CHO intermediate. Acyl derivatives R⁵CH₂COW may be prepared by activation of the R⁵-CH₂-ester. R⁵-V-CH₂-halides such as bromides may be prepared from the alcohol R⁵-V'-CH₂OH by reaction with phosphorus tribromide in DCM/triethylamine. R⁵-V'-CH₂-W derivatives such as methanesulphonyl derivatives may be prepared from the alcohol R⁵-V'-CH₂OH by reaction with methane sulphonyl chloride. R⁵V'SO₂W derivatives may be prepared by a route analogous to that of Ahmed El Hadri *et al, J. Heterocyclic Chem.,* 1993, 30(3), 631. Thus compounds of formula R⁵CH₂SO₂OH may be prepared by reacting the corresponding R⁵CH₃ compound with N-bromosuccinimide, followed by treatment with sodium sulfite. The leaving group W may be converted to another leaving group W, e.g. a halogen group, by conventional methods. The R⁵-V'-U- derivatives may be prepared by various conventional strategies. For example the homologous aldehyde R⁵-CHO may be treated with trimethylsulfonium methylsulfate in basic conditions, to give an epoxide intermediate (see *Synth. Commun.,* 749, **1985**) which is then treated with lewis acid, such as trifluoroboron etherate or diethyl etherate, to provide the desired aldehyde (see *JOC,* 1720, **1999**). The aldehyde R⁵-CHO could also be treated with an appropriate phosphonium salt, such as (methoxymethyl)triphenylphosphonium chloride, in a Wittig fashion reaction. The resulting enol ether can readily be hydrolysed to homologous aldehydes *(Chem. Ber.,* 2514, **1962**). R⁵-COW derivatives can be converted to the aldehyde R⁵-V'-CHO in several steps (see *JACS,* 1325, 1986). The R⁵COCH₂-halide derivatives may be prepared by standard methods from the R⁵CO₂H derivative, for example, by acid chloride formation, conversion to the alpha-diazoketone with diazomethane and reaction with a halogen acid to provide the halomethylketone. Vinyl derivatives R⁵-CH=CH₂ may be prepared from the corresponding diethylaminoethyl derivative by quatemisation with dimethyl sulphate and heating, resulting in elimination of the charged amino group. The diethylaminoethyl derivative may be prepared from another ethyl derivative eg the hydroxyethyl by conventional amine formation. Alternatively, it may be prepared from a methyl derivative by condensation with diethylamine and formaldehyde.

Where R⁵ is an optionally substituted benzoimidazol-2-yl group, the compound of formula (V) where R⁴' is R⁴ may be obtained by reacting N-1 protected piperazine with acrylnitrile, converting the resulting R^{4'} 2-cyanoethyl group with partial hydrolysis to give the 2-ethoxycarbonimidoylethyl group which can then be condensed with an appropriately substituted 1,2-diaminobenzene to give the required benzoimidazol-2-yl group.

R⁵-H heterocycles are commercially available or may be prepared by conventional methods.

Conversions of R^{1a'}, R^{1'}, R^{3'} and R^{4'} may be carried out on the intermediates of formulae (IV), (V) and (Vb) prior to their reaction to produce compounds of formula (I) in the same way as described above for conversions after their reaction.

The pharmaceutical compositions of the invention include those in a form adapted for oral, topical or parenteral use and may be used for the treatment of bacterial infection in mammals including humans.

The antibiotic compounds according to the invention may be formulated for administration in any convenient way for use in human or veterinary medicine, by analogy with other antibiotics.

The composition may be formulated for administration by any route, such as oral, topical or parenteral. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No toxicological effects are indicated when a compound of formula (I) or a pharmaceutically acceptable salt or *in vivo* hydrolysable ester thereof is administered in the above-mentioned dosage range.

The compound of formula (I) may be the sole therapeutic agent in the compositions of the invention or a combination with other antibiotics or with a β-lactamase inhibitor may be employed.

Compounds of formula (I) are active against a wide range of organisms including both Gram-negative and Gram-positive organisms.

The following examples illustrate the preparation of certain compounds of formula (I) and the activity of certain compounds of formula (I) against various bacterial organisms.

### Examples

### Example 1 2-(2-{4-[(R)-2-Hydroxy-2-(6-methoxy-quinolin-4-yl)-ethyl]-piperazin-1-yl}-ethyl)-isoindole-1,3-dione dioxalate

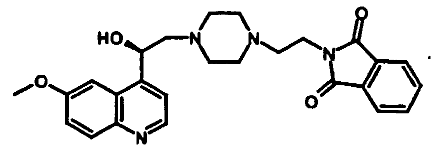

### (a) 6-Methoxyquinoline-4-carboxylic acid

The title compound was prepared by modification of the procedure described by W.E. Daering and J.D. Chanley, *J. Amer. Chem. Soc.,* 1946, **68**, 586. A mixture of Quinine (225g, 0.70 mol), tert-butanol (1 litre) and water (10 ml) was treated with potassium *tert-*butoxide (170g, 1.5 mol). The mixture was stirred at 30°C, while air was bubbled through for 3 days. The mixture was diluted with diethyl ether and water and the layers separated. The aqueous phase was extracted with ethyl acetate. The combined diethyl ether and ethyl acetate extracts were dried over magnesium sulfate and evaporated to give recovered starting material (approximately 100g). The aqueous phase was acidified to pH5 with 5M hydrochloric acid. The precipitate was collected by filtration, washed with water and methanol, then dried to give 6-methoxyquinoline-4-carboxylic acid as a yellow solid (64.6g, 46%).
δH (d-6 DMSO), 6.23-5.95 (1H, m), 5.34-5.06 (2H, m), 3.37-2.92 (5H, m), 2.70 (1H, m), 2.38-2.15 (3H, m), 1.94-1.52 (2H, m).

### (b) (R)-2-(6-Methoxyquinolin-4-yl)oxirane

A solution of 6-methoxyquinoline-4-carboxylic acid (Example 1(a)) (10g) in dichloromethane was heated under reflux with oxalyl chloride (5ml) and N,N'-dimethylformamide (2 drops) for 1 hour and evaporated to dryness. The residue, in dichloromethane (100ml) was treated with a 2M solution of trimethylsilyldiazomethane in hexane (50ml) and stirred at room temperature for 18 hours. 5M Hydrochloric acid (150ml) was added and the solution was stirred at room temperature for 3 hours. It was basified with sodium carbonate solution, extracted with ethyl acetate and chromatographed on silica gel eluting with ethyl acetate-hexane to give the chloromethyl ketone (4.2g). A batch of the chloromethyl ketone (20g) was reduced with (+)-B-chlorodiisopinocamphenylborane (40g) in dichloromethane (400ml) at room temperature for 18 hours followed by treatment with diethanolamine (30g) for 3 hours. The product was chromatographed on silica gel eluting with ethyl acetate-hexane to give the chloroalcohol (16.8g), which was dissolved in tetrahydrofuran (100 ml) and reacted with sodium hydroxide (2.6g) in water (13ml) for 1.5 hours. The reaction mixture was evaporated to dryness and chromatographed on silica gel eluting with ethyl acetate/hexane to give the title compound as a solid (10.4 g) (84% ee by chiral HPLC). Recrystallisation from ether-pentane gave mother-liquor (7.0 g) (90% ee).
MS (+ve ion electrospray) m/z 202 (MH+)
The absolute stereochemistry was defined to be (*R*) by an NMR study on the Mosher's esters derived from the product obtained by reaction with 1-*tert*-butylpiperazine.

### (c) 4-[(R)-2-Hydroxy-2-(6-methoxy-quinolin-4-yl)-ethyl]-piperazine-1-carboxylic acid tert-butyl ester

(*R*)-2-(6-Methoxyquinolin-4-yl)oxirane (Example 1(b)) (4.30g, 21.37mmol) was dissolved in acetonitrile (30mL). To the solution was added piperazine-1-carboxylic acid *tert-*butyl ester (7.17g, 38.47mmol) and lithium perchlorate (2.27g, 106.4mmol). The resulting slurry was stirred at room temperature for 10 hours and then concentrated *in vacuo.* The residue was partitioned between ethyl acetate and water and the organic phase dried over magnesium sulfate. Concentration *in vacuo* afforded a colourless oil which was subjected to purification by column chromatography on silica gel using a dichloromethane/methanol gradient. This provided the desired compound as a colourless oil (7.65g, 92%).
MS (APCI+) m/z 388 (MH+).

### (d) (R)-1-(6-Methoxy-quinolin-4-yl)-2-piperazin-1-yl-ethanol

4-[(*R*)-2-Hydroxy-2-(6-methoxy-quinolin-4-yl)-ethyl]-piperazine-1-carboxylic acid *tert-*butyl ester (Example 1 (c)) (3.50g) was dissolved in dichloromethane (10mL) and trifluoroacetic acid (10mL) was added. The resulting solution was stirred at room temperature for 5 hours and then concentrated *in vacuo.* The residue was purified on silica gel, eluting with methanol and dichloromethane. This provided the desired compound which was used without further purification.
MS (APCI+) m/z 288 (MH+).

### (e) Title compound

(*R*)-1-(6-Methoxy-quinolin-4-yl)-2-piperazin-1-yl-ethartol (Example 1(d), crude from above) was dissolved in N,N'-dimethylformamide (3mL). To the solution was added potassium carbonate (111 mg, 0.807mmol) and 2-(2-bromo-ethyl)-isoindole-1,3-dione (205mg, 0.807mmol). Stirring at room temperature was continued for 3 hours and then the mixture was concentrated *in vacuo.* The residue was purified by column chromatography on silica gel to provide the desired product as a colourless oil (35mg, 11%).
δH (CD₃OD, 250MHz), 8.45-8.43 (1H, d), 7.73-7.67 (2H, m), 7.56-7.09 (6H, m), 5.42-5.38 (1H, dd), 4.25-4.18 (2H, m), 3.74 (3H, s), 3.81-3.58, 2.62-2.41 (12H, m).
MS (APCI+) m/z 461 (MH+).
A solution of the oil (20mg) in dichloromethane (1mL) was added to oxalic acid (8mg) in diethyl ether (10mL) to generate the dioxalate salt. The title compound was isolated by centrifugation, washing with diethyl ether and subsequent drying *in vacuo.*

### Example 2 (R)-2-{4-[2-(4-Fluoro-1H-benzoimidazol-2-yl)-ethyl]-piperazin-1-yl}-1-(6-methoxy-quinolin-4-yl)-ethanol

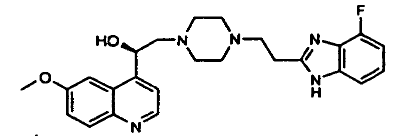

### (a) 4-(2-Cyano-ethyl)-piperazine-1-carboxylic acid 9H-fluoren-9-ylmethyl ester.

A mixture of 5.17 g (15 mmol) of 9-fluorenylmethyloxylcarbonyl piperazine hydrochloride (RN 215190-22-0), 0.987 ml (15 mmol) of acrylonitrile, 2.081 ml (15 mmol) of triethylamine and 15 ml of isopropylalcohol was stirred 5 min at 40°C followed by 3 h at room temperature. The white precipitate was filtered, washed with diethylether, then dissolved in ethyl acetate. The organic phase was washed with water, dried over magnesium sulphate and concentrated to dryness. The residue was suspended in a small amount of diethyl ether, filtered and dried to afford 3.1 g (yield: 57%) of the title compounds as white crystals.
δH (CDCl3, 200MHz): 7.77(2H, d), 7.57(2H, d), 7.50-7.21(4H, m), 4.45(2H, d), 4.24(1H, t), 3.49(4H, m), 2.74(2H, t), 2.51(2H, t), 2.43(4H, m).

### (b) 4-(2-Ethoxycarbonimidoyl-ethyl)-piperazine-1-carboxylic acid 9H-fluoren-9-ylmethyl ester, dihydrochloride

A solution of 1.48 g (4.09 mmol) of 4-(2-cyano-ethyl)-piperazine-1-carboxylic acid 9H-fluoren-9-ylmethyl ester (Example 2(a)) and 239 microliters of ethanol (4.09 mmol) in 5 ml of CHCl₃ was saturated, at O°C, by a stream of gaseous hydrochloric acid. The mixture was left overnight in a fridge. The solution was concentrated *in vacuo* and the white residue (1.97 g) used without further purification in the next step.

### (c) 4-[2-(4-Fluoro-1H-benzoimidazol-2-yl)-ethyl]-piperazine-1-carboxylic acid 9H-fluoren-9-ylmethyl ester

A mixture of 1.97 g (approx. 4 mmol) of crude 4-(2-ethoxycarbonimidoyl-ethyl)-piperazine-1-carboxylic acid 9H-fluoren-9-ylmethyl ester dihydrochloride (Example 2(b)), 0.52 g (4.09 mmol) of 3-fluoro-1,2-diaminobenzene (RN 18645-88-0) and 8 ml of CHCl₃ was heated to reflux for 1 h. The mixture was diluted with MDC and neutralised with an aqueous solution of potassium carbonate. The organic phase was washed with water, dried over magnesium sulphate and concentrated. The residue was purified by flash chromatography on silica gel (eluent : first AcOEt, then AcOEt/MeOH : 98/2) to afford 1.15 g (yield 60% over two steps) of the title compound as off-white crystals.
δH (CDCl3, 200MHz): 10.98(1H,br), 7.78(2H, d), 7.59(2H, d), 7.50-7.22(5H, m), 7.16(1H, m), 6.94(1H, dd), 4.50(2H, d), 4.26(1H, t), 3.58(4H, m), 3.17(2H, t), 2.85(2H, t), 2.55(4H, m).

### (d) 4-Fluoro-2-(2-piperazin-1-yl-ethyl)-1H-benzoimidazole

A mixture of 0.49 g (1.04 mmol) of 4-[2-(4-fluoro-1H-benzoimidazol-2-yl)-ethyl]-piperazine-1-carboxylic acid 9H-fluoren-9-ylmethyl ester (Example 2(c)), 156 ul (1.6 mmol) of piperidine and 27 ml of acetonitrile was stirred at room temperature for 21 h. After concentration of the solvent, the residue was purified by flash chromatography over 25 g silica gel (eluent : first CH₂Cl₂/MeOH : 90/10, then 80/20). The most polar fractions were pooled and concentrated affording 0.167 g (yield 65%) of the title compound as a white solid.
δH (CDCl3, 200MHz): 7.30(1H, d), 7.12(1H, m), 6.92(1H, dd), 3.14(2H, t), 3.02(4H, m), 2.82(2H, t), 2.62(4H, m), 1.90(2H, br).

### (e) Title compound

A mixture of 160 mg (0.644 mmol) of 4-fluoro-2-(2-piperazin-1-yl-ethyl)-1H-benzoimidazole (Example 2(d)), 118 mg (0.586 mmol) of (*R*)-2-(6-methoxyquinoloin-4-yl)oxirane (Example 1(b)), 62 mg (0.586 mmol) of anhydrous lithium perchlorate and 1.6 ml of anhydrous acetonitrile were stirred for 24 h at room temperature. The solvent was concentrated and the residue dissolved in 15 ml AcOEt. The organic phase was washed with brine, dried over magnesium sulphate and concentrated. The residue was purified by flash chromatography over 25 g silica gel (eluent: CH₂Cl₂/MeOH : 95/5) affording 171 mg (yield 59%) of the title compound as the free base.
A solution of 36 mg (0.396 mmol) of oxalic acid in 2 ml of acetone was added to a solution of 89 mg (0.198 mmol) of the base described above in 2 ml of acetone. The precipitate was filtered, washed with a small amount of diethyl ether and dried to afford 76 mg of the title compound as the oxalate salt as off-white crystals. m.p. = 291-293 °C.
δH (d-6 DMSO, 200MHz): 8.77(1H, d), 7.75(1H, d), 7.62(1H, d), 7.51-7.37(2H, m), 7.32(1H, d), 7.15(1H, m), 6.95(1H, m), 5.71(1H, m), 4.32(5H, br), 3.95(3H, s), 3.35-2.80(14H, m).

### Example 3 4-[2-(4-Fluoro-1H-benzoimidazol-2-yl)-ethyl]-piperazine-1-carboxylic acid (6-methoxy-[1,5]naphthyridin-4-yl)-amide

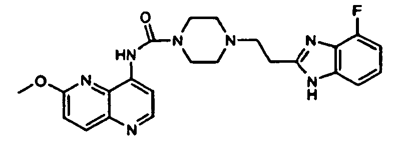

A mixture of 150 mg (0.856 mmol) of 4-amino-6-methoxy-[1,5]naphthyridine (WO00/21948, Example 3(e)), 115 mg (0.942 mmol) of 4-dimethylaminopyridine (DMAP), 194 mg (1.2 mmol) of carbonyl diimidazole and 4 ml of chloroform were stirred for 6 hours. The solvent was concentrated and the residue dissolved in 2 ml of dry DMF. Then, 213 mg (0.856 mmol of 4-fluoro-2-(2-piperazin-1-yl-ethyl)-1H-benzoimidazole (Example 2(d)) were added and the mixture was heated at 70°C for 18 h. The solvent was concentrated *in vacuo* and the residue was dissolved in ethyl acetate and the aqueous phase was washed with water. On standing at room temperature 270 mg of the title compound free base precipitated slowly (yield 70%).
δH (d-6 DMSO, 200MHz): 9.09(1H, s), 8.61(1H, d), 8.21(2H, m), 7.29(2H, d), 7.10(1H, m), 6.93(1H, dd), 4.08(3H, s), 3.58(4H, m), 3.20(1H, br), 3.04(2H, t), 2.85(2H, t), 2.58(4H, m).
The free base described above (100 mg) was salified following the process of example 2(e) to afford 129 mg (yield 92%) of the title compound as the oxalate salt as off-white crystals. m.p. = 263-265°C.
δH (d-6 DMSO, 200MHz): 9.16(1H, s), 8.63(1H, d), 8.32-8.16(2H, m), 7.40-7.24(2H, m), 7.13(1H, m), 6.98(1H, dd), 4.57(5H, br), 4.10(3H, s), 3.73(4H, m), 3.41-3.14(4H, m), 3.03(4H, m).

### Example 4 (R)-2-{4-[2-(5-Fluoro-1H-benzoimidazol-2-yl)-ethyl]-piperazin-1-yl}-1-(6-methoxy-quinolin-4-yl)-ethanol

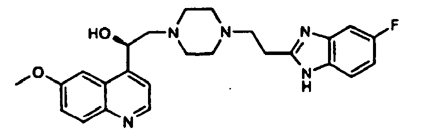

### (a) 4-[2-(5-Fluoro-1H-benzoimidazol-2-yl)-ethyl]-piperazine-1-carboxylic acid 9H-fluoren-9-ylmethyl ester

A mixture of 1.15 g (approx. 2.4 mmol) of crude 4-(2-ethoxycarbonimidoyl-ethyl)-piperazine-1-carboxylic acid 9H-fluoren-9-ylmethyl ester dihydrochloride (Example 2(b)), 0.30 g (2.4 mmol) of 4-fluoro-1,2-diaminobenzene (RN 367-31-7) and 4.8 ml of CHCl₃ was heated to reflux for 5 h. The mixture was diluted with dichloromethane (MDC) and neutralised with an aqueous solution of potassium carbonate. The organic phase was washed with water, dried over magnesium sulphate and concentrated. The residue was purified by flash chromatography on silica gel (eluent : AcOEt/MeOH : 95/5) to afford 0.49 g (yield 43%) of the title compound as light brown crystals.
δH (CDCl3, 200MHz): 7.77(2H, d), 7.58(2H, d), 7.51-7.18(6H, m), 6.98(1H, td), 5.42(1H, br), 4.51(2H, d), 4.25(1H, t), 3.59(4H, m), 3.17(2H, d), 2.89(2H, t), 2.56(4H, m).

### (b) 5-Fluoro-2-(2-piperazin-1-yl-ethyl)-1H-benzoimidazole

Starting with Example 4(a) and following the procedure of Example 2d afforded the desired compound as white crystals.
δH (CDC13, 200MHz): 7.45(1H, dd), 7.23(1H, dd), 6.96(1H, td), 3.30(2H, br), 3.10(2H, t), 3.02(4H, m), 2.81 (2H, t), 2.60(4H, m).

### (c) Title compound.

Starting from 100 mg (0.403 mmol) of 5-fluoro-2-(2-piperazin-1-yl-ethyl)-1H-benzoimidazole (Example 4(b)) and following the procedure of example 2(e) afforded 120 mg (yield 0%) of the title compound free base.
δH (CDCl3, 200MHz): 8.78(1H, d), 8.06(1H, d), 7.65(1H, d), 7.53-7.35(2H, m), 7.30-7.17(2H, m), 6.96(1H, td), 5.52(1H, dd), 3.94(3H, s), 3.62(2H, br), 3.17(2H, t), 3.12-2.58(12H, m).
The base described above (120 mg) was salified following the process of example 2(e) to afford 120 mg (yield 7 1 %) of the title compound as the oxalate salt as yellowish crystals. m.p. 291-293.
δH (d-6 DMSO, 200MHz): 8.76(1H, d), 7.97(1H, d), 7.62(1H, d), 7.58-7.26(4H, m), 7.00(1H, td), 5.70(1H, m), 3.93(3H, s), 3.80(6H, br), 3.33-2.80(14H, m).

### Example 5 4-[2-(2,3-Dihydro-benzo[1,4]dioxin-6-yl)-ethyl]-piperazine-1-carboxylic acid (6-methoxy-[1,5]naphthyridin-4-yl)-amide, dioxalate.

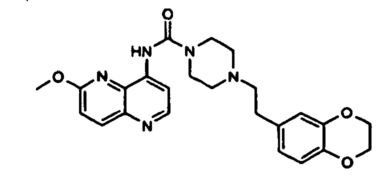

### (a) 6-Oxiranyl-2,3-dihydro-benzo[1,4]dioxine

A mixture of 1,4-benzodioxanecarboxaldehyde [RN 29668-44-8] (1.5 g, 9.14 mmol), trimethylsulfonium methyl sulphate (2.24 g, 11.9 mmol), 35 ml of methylene chloride and 4.6 ml of 50% aqueous sodium hydrochloride were vigorously stirred for 70 hours. The mixture was diluted with 20 ml of water and 40 ml of diethyl ether and the layers separated. The aqueous phase was extracted with diethyl ether. The combined organic phases were washed with 20 ml of water, 20 ml of brine then dried over magnesium sulphate and evaporated to give crude 6-oxiranyl-2,3-dihydro-benzo[1,4]dioxine (1.66 g, quantitative).
δH (CDCl3, 200MHz): 6.88-6.72(3H, m), 4.25(4H, s), 3.76(1H, dd), 3.10(1H, dd), 2.77(1H, dd).

### (b) (2,3-Dihydro-benzo[1,4]dioxin-6-yl)-acetaldehyde.

A solution of crude 6-oxiranyl-2,3-dihydro-benzo[1,4]dioxine (Example 5(a), 200 mg, 1.12 mmol) in 20 ml anhydrous diethyl ether was cooled at - 10°C. A 10% solution of boron trifluoride diethyl etherate in diethyl ether (160 ul) was then added and the mixture was stirred for 10 minutes at -10°C. The reaction was quenched at this temperature by addition of 10 ml of a saturated aqueous solution of sodium bicarbonate. The mixture was extracted twice with diethyl ether. The combined organic phases were washed with 10 ml of water, 10 ml of brine then dried over magnesium sulphate and evaporated to give crude (2,3-dihydro-benzo[1,4]dioxin-6-yl)-acetaldehyde (191 mg, 95%) used without further purification in the next step.
δH (CDCl3, 200MHz): 9.70(1H, t), 6.95-6.62(3H, m), 4.26(4H, s), 3.56(2H, d).

### (c) 4-(6-Methoxy-[1,5]naphthyridin-4-ylcarbamoyl)-piperazine-1-carboxylic acid tert-butyl ester

A mixture of 4-amino-6-methoxy-[1,5]naphthyridine (1.5 g), 4-dimethylaminopyridine (DMAP) (1.2 g) and carbonyl diimidazole (1.95 g) in chloroform (45 ml) was stirred for 5 hours. The solvent was evaporated and the residue dissolved in dry DMF (20 ml) and piperazine-1-carboxylic acid tert-butyl ester (1.6 g) was added and the solution was heated at 70°C overnight. Water was added and the mixture was extracted with ethyl acetate, dried (magnesium sulfate,) and concentrated to give a solid (1.04 g).

### (d) Piperazine-1-carboxylic acid (6-methoxy-[1,5]naphthyridin-4-yl)-amide

The ester (5c) (1.0 g) in dichloromethane (15 ml) and trifluoroacetic acid (8 ml) was stirred at room temperature for 4 hours and evaporated to dryness. Water was added and excess 15% sodium hydroxide solution and the mixture was extracted with dichloromethane, dried (magnesium sulfate), and evaporated to give a solid (0.4 g)

### (e) Title compound.

A mixture of (2,3-dihydro-benzo[1,4]dioxin-6-yl)-acetaldehyde (Example 5(b), 138 mg, 0.78 mmol), piperazine-1-carboxylic acid (6-methoxy-[1,5]naphthyridin-4-yl)-amide (Example 5(d) 200 mg, 0.7 mmol), acetic acid (3 drops) and methanol (20 ml) was stirred at room temperature for 0.5 hour, then sodium cyanoborohydride (35 mg, 0.56 mmol) was added and the mixture was stirred at room temperature for 48 hours. The solvent was concentrated and the residue was treated with 0.1 N NaOH (20 ml) then extracted with methylene chloride. The solution was dried over magnesium sulphate and concentrated to dryness. The residue was chromatographed on silica gel eluting with methylene chloride-methanol-ammonia (99-1-0.6) to give the title compound as the free base (184 mg, 41%).
δH(d-6 DMSO, 200MHz): 9.10(1H, br), 8.61(1H, d), 8.24(1H, d), 8.21(1H, d), 7.30(1H, d), 6.80-6.60(3H, m), 4.20(4H, s,br), 4.08(3H, s), 3.56(4H, m), 2.63(2H, in), 2.60-2.44(6H,m).
A suspension of the solid obtained above (134 mg, 0.29 mmol) in acetone (15 ml) was treated wit a solution of oxalic acid (74 mg, 0.59 mmol) in acetone (2 ml) to give, after concentration the title compound (208 mg). mp : 158-159°C.
δH(d-6 DMSO, 200MHz): 9.19(1H, br), 8.63(1H,d), 8.26(1H, d), 8.21(1H, d), 7.31(1H,d), 6.87-6.64(3H, m), 5.60(4H, br), 4.21(4H,s,br), 4.10(3H, s), 3.77(4H, m), 3.26-3.00(6H, m), 2.87(2H, m).

### Example 6 4-(2-Benzo[1,3]dioxol-5-yl-ethyl)-piperazine-1-carboxylic acid (6-methoxy-[1,5]naphthyridin-4-yl)-amide, dioxalate.

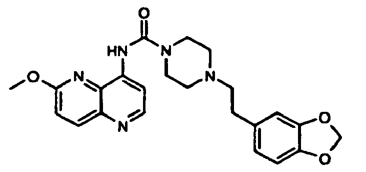

A mixture of benzo[1,3]dioxol-5-yl-acetaldehyde [RN 6543-34-6] (114 mg, 0.7 mmol), piperazine-1-carboxylic acid (6-methoxy-[1,5]naphthyridin-4-yl)-amide (Example 5(d) 200 mg, 0.7 mmol), acetic acid (3 drops) and methanol (20 ml) was stirred at room temperature for a few minutes, then sodium cyanoborohydride (35 mg, 0.6 mmol) was added and the mixture was stirred at room temperature for 2 hours. The solvent was concentrated and the residue was treated with 0.1 N NaOH (20 ml) then extracted twice with methylene chloride (2x20 ml). The organic phase was dried over magnesium sulphate and concentrated to dryness. The residue was chromatographed on silica gel eluting with methylene chloride-methanol-ammonia (99-1-0.6) to give the title compound as the free base (169 mg, 55%).
δH(d-6 DMSO, 200MHz): 9.10(1H, br), 8.61(1H, d), 8.30-8.16(2H, m), 7.7.30(1H, d), 6.88-6.76(2H, m),6.69(1H, dd), 5.96(2H, s), 4.08(3H, s), 3.56(4H, m), 2.69(2H, m), 2.62-2.45(6H, m).
A suspension of the solid obtained above (120 mg, 0.277 mmol) in acetone (15 ml) was treated with a solution of oxalic acid (70 mg, 0.555 mmol) in acetone (2 ml) to give, after concentration, the title compound. mp : 208-210°C.
δH(d-6 DMSO, 200MHz):9.18(1H, br), 8.63(1H, d), 8.26(1H, d), 8.21(1H, d), 7.31(1H, d), 6.92-6.82(2H, m), 6.73(1H, dd), 5.98(2H, s), 4.15(4H, br), 4.10(3H, s), 3.76(4H, m), 3.21-2.97(6H, m), 2.87(2H, m).

### Example 7 4-[2-(3-Oxo-3,4-dihydro-2H-benzo[1,4]thiazin-6-yl)-ethyl]-piperazine-1-carboxylic acid (6-methoxy-[1,5]naphthyridin-4-yl)-amide.

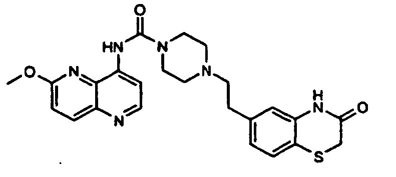

### (a) 2-(4-Fluoro-3-nitro-phenyl)-ethanol.

A solution of (4-fluoro-3-nitro-phenyl)-acetic acid (3 g, 15 mmol) in dimethoxyethane (15 ml) was cooled at -15°C. 4-Methylmorpholine (1.53 g, 15 mmol) was then added dropwise followed by isobutylchloroformate (2.15 g, 15.7 mmol) also added dropwise. After a few minutes the precipitate of N-methylmorpholine hydrochloride was filtered off and washed three times with dimethoxyethane (3x5 ml). The solution was transferred to a 11 vial and cooled with an ice-salt bath and treated with a solution of sodium borohydride (0.855 g, 23 mmol) in water (7 ml). A discharge of hydrogen was observed and the solution becoming orange was then diluted with water (375 ml). The solution was extracted three time with ethyl acetate. The organic phase was washed with water, dried over magnesium sulphate and concentrated to dryness. The residue was chromatographed on silica gel eluting with heptane-ethyl acetate (1-1) to give 2-(4-fluoro-3-nitro-phenyl)-ethanol (1.1 g, 39.6%).
δH(CDCl3, 200MHz): 7.95(1H, dd), 7.64-7.46(1H, m), 7.23(1H, dd), 3.92(2H, t), 2.92(2H, t), 1.58(1H, br).

### (b) [4-(2-Hydroxy-ethyl)-2-nitro-phenylsulfanyl]-acetic acid.

A mixture of 2-(4-fluoro-3-nitro-phenyl)-ethanol (Example 7(a),1.1 g, 6 mmol), mercaptoacetic acid (0.61 g, 6.6 mmol), potassium carbonate (2.8 g, 20 mmol) and 12 ml DMF was heated at 70°C for 4 hours. The mixture was poured onto 200 ml of water and the aqueous phase was washed with ethyl acetate. The aqueous phase was then acidified with 1N HCl and extracted three times with 100 ml of methylene chloride. The extracts were dried over magnesium sulphate and evaporated to give [4-(2-hydroxy-ethyl)-2-nitro-phenylsulfanyl]-acetic acid (0.92 g, 60%).
δH (d-6 DMSO, 200MHz): 8.07(1H, d), 7.60(1H, dd), 7.50(1H, d), 3.92(2H, s), 3.63(2H, t), 3.50(2H, br), 2.79(2H, t).

### (c) 6-(2-Hydroxy-ethyl)-4H-benzo[1,4]thiazin-3-one.

A mixture of iron powder (1.24 g, 22 mmol), sodium chloride (1.24 g, 21 mmol) and 25 ml of 50% aqueous ethanol was heated under reflux for 15 minutes. A solution of crude [4-(2-hydroxy-ethyl)-2-nitro-phenylsulfanyl]-acetic acid (Example 7(b), 0.92 g, 3.6 mmol) in 5 ml of ethanol was then added quickly and the reaction mixture was heated under reflux for 2 hours. The mixture was filtered on Clarcel® and the filtration cake was rinsed with a small amount of methanol. The alcoholic solution was evaporated to dryness. The residue was dissolved in water and acidified to pH 5 with 1N hydrochloric acid. The precipitate was collected by filtration, washed with water, then dried to give 6-(2-hydroxy-ethyl)-4H-benzo[1,4]thiazin-3-one (0.35 g, 46%).
δH (d-6 DMSO, 200MHz): 10.48(1H, br), 7.20(1H, d), 6.92-6.87(2H, m), 4.65(1H, t), 3.56(2H, q), 3.42(2H, s), 2.64(2H,t).

### (d) Methanesulfonic acid 2-(3-oxo-3,4-dihydro-2H-benzo[1,4]thiazin-6-yl)-ethyl ester

A solution of 6-(2-hydroxy-ethyl)-4H-benzo[1,4]thiazin-3-one (Example 7(c), 0.34 g, 1.67 mmol) and 250 ul of triethylamine (0.19 g, 1.8 mmol) in 5 ml of methylene chloride stabilised with amylene was cooled to -15°C by mean of an acetone/ice bath and treated dropwise by 135 ul of methanesulfonyl chloride (1.75 mmol). The ice bath was removed and the mixture stirred 15 hours at room temperature. The organic phase was separated, washed with water, dried over magnesium sulphate and evaporated. The crude product was chromatographed on silica gel eluting with ethyl acetate-heptane (2-1) to give the desired methanesulfonic acid 2-(3-oxo-3,4-dihydro-2H-benzo[1,4]thiazin-6-yl)-ethyl ester (0.36 g, 75%).
δH (d-6 DMSO, 200MHz): 10.55(1H, br), 7.27(1H, d), 6.92(1H, dd), 6.87(1H, d), 4.37(2H, t), 3.45(2H, s), 3.13(3H, s), 2.93(2H, t).

### (e) Title compound.

A solution of piperazine-1-carboxylic acid (6-methoxy-[1,5]naphthyridin-4-yl)-amide (Example 5(d) 250 mg, 0.87 mmol), methanesulfonic acid 2-(3-oxo-3,4-dihydro-2H-benzo[1,4]thiazin-6-yl)-ethyl ester (Example 7(d), 250 mg, 0.87 mmol), triethylamine (135 ul, 0.97 mmol) and acetonitrile (15 ml) was heated under reflux for 18 hours. The mixture was diluted with water. The precipitate was collected by filtration, washed with water then with a small amount of acetonitrile and dried. The crude compound was chromatographed on silica gel eluting with methylene chloride-methanol (96-4) to give the title compound as a white solid (85 mg, 20%). mp : 248-250°C.
δH(d-6 DMSO, 200MHz): 10.51(1H, br), 9.11(1H, br), 8.61(1H, d), 8.30-8.18(2H, m), 7.30-1H, d), 7.21(1H, d), 6.92-6.82(2H, m), 4.08(3H, s), 3.57(4H, m), 3.43(2H,s), 2.69(2H, m), 2.64-2.46(6H, m).

### Example 8 4-(2-Quinoxalin-2-yl-ethyl)-piperazine-1-carboxylic acid (6-methoxy-[1,5]naphthyridin-4-yl)-amide.

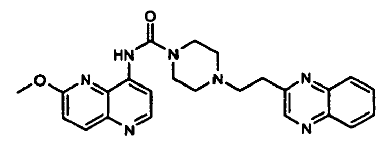

### (a) Diethyl-(2-quinoxalin-2-yl-ethyl)-amine

A solution of diethylamine hydrochloride (3.82 g, 35 mmol) and triethylamine (0.23 ml) in ethanol (1.75 ml) and water (1.75 ml) was added dropwise, at 60°C, to a mixture of 2-methyl-quinoxaline (5 g, 35 mmol), formaldehyde (2.81 g of 37% solution, 35 mmol), triethylamine (0.17 ml) and ethanol (4.6 ml). If necessary the pH was adjusted to 7-7.5 by mean of diluted HCl. Stirring was maintained 16 hours at 60°C. The solvent was concentrated, the residue was diluted with water and extracted with diethyl ether to remove the un-reacted quinoxaline. The aqueous phase was made alkaline with 20% aqueous sodium hydroxide (10 ml) and extracted with ethyl acetate. The organic phase was dried over magnesium sulphate and concentrated. The solid was then chromatographed on silica gel eluting with methylene chloride-methanol (first 95-5 then 90-10 and finally 90-10 plus 0.1% of ammonia) to give diethyl-(2-quinoxalin-2-yl-ethyl)-amine (2.67 g, 37%).
δH(CDCl3, 200MHz): 8.78(1H, s), 8.05(2H, m), 7.72(2H, m), 3.17(2H, m), 3.00(2H, m), 2.64(4H, q), 1.05(6H, t).

### (b) 2-Vinyl-quinoxaline.

Dimethyl sulphate (1.07 ml, 11.3 mmol) was added dropwise, while maintaining the mixture temperature below 40°C, to a solution of diethyl-(2-quinoxalin-2-yl-ethyl)-amine (Example 8(a), 2.6g 11.3 mmol) in ethanol (3 ml). The thick mixture was diluted with water (15 ml), triethylamine (1.13 ml) and heated 2 hours on a water bath. After cooling the mixture was extracted with diethyl ether. The organic phase was dried over magnesium sulphate, concentrated to dryness and the residue was chromatographed on silica gel eluting with heptane-ethyl acetate (3-1) to give) 2-vinyl-quinoxaline (1.16 g, 65.5%).
δH(CDCl3, 200MHz): 9.00(1H, s), 8.07(2H, m), 7.75(2H, m), 7.05(1H, dd), 6.48(1H, dd), 5.80(1H, dd).

### (c) Title compound.

A mixture of piperazine-1-carboxylic acid (6-methoxy-[1,5]naphthyridin-4-yl)-amide (Example 5(d) 0.403 g, 1.4mmol), 2-vinyl-quinoxaline (Example 8(b), 0.22 g, 1.4 mmol), acetic acid (0.104 ml) and ethanol (5 ml) was heated under reflux for 6 hours. The mixture was then diluted with ethyl acetate and made basic with aqueous sodium hydroxide. The precipitate was collected by filtration and washed with water and a small amount of ethyl acetate. The solid was then dissolved in hot methanol and the insoluble residue, corresponding to an impurity, was discarded. The solution was concentrated to dryness and the residue crystallized in acetonitrile to give the title compound (220 mg, 35%). mp : 138-140°C.
δH(d6-DMSO,200MHz): 9.08(1H, br), 8.94(1H, s), 8.60(1H, d), 8.32-8.14(2H, m), 8.13-7.97(2H, m), 7.91-7.72(2H, m), 7.29(1H, d), 4.07(3H, s), 3.54(4H, m), 3.21(2H, t), 2.88(2H, t), 2.59(4H, m).

### Example 9 4-(2-Benzo[1,2,5]thiadiazol-5-yl-ethyl)-piperazine-1-carboxylic acid (6-methoxy-[1,5]naphthyridin-4-yl)-amide.

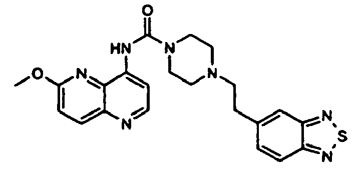

### (a) Benzo[1,2,5]thiadiazol-5-yl-acetonitrile [RN 89899-11-6].

A mixture of 5-bromomethyl-benzo[1,2,5]thiadiazole [RN 65858-50-6] (7 g, 30 mmol), 50 ml of ethanol and a solution of potassium cyanide (2.3 g, 36 mmol) in 25 ml of water was stirred at 50°C for 15 hours. The alcoholic solution was concentrated and the residue suspended in water. The solid was collected by filtration, washed with water and dried *in vacuo* to give benzo[1,2,5]thiadiazol-5-yl-acetonitrile (2.8 g, 47%)
δH(CDCl3, 200MHz): 8.12-7.97(2H, m), 7.53(1H, dd), 3.96(2H, s).

### (b) Benzo[1,2,5]thiadiazol-5-yl-acetic acid [RN 55937-37-6]

A suspension of benzo[1,2,5]thiadiazol-5-yl-acetonitrile (Example 9(a), 2.8 g, 14 mmol) in 6N aqueous hydrochloric acid (70 ml) was heated under reflux for 4 hours. The mixture was cooled at room temperature and diluted with more water. The precipitate was collected by filtration, washed with water and dried *in vacuo* to give crude benzo[1,2,5]thiadiazol-5-yl-acetic acid (2.8 g, 100%).
δH(DMSO-d6, 200MHz): 12.58(1H, br), 8.03(1H, d), 7.97(1H, d), 7.65(1H, dd), 3.86(2H, s).

### (c) 2-Benzo[1,2,5]thiadiazol-5-yl-ethanol.

Borane methylsulfide (1.6 ml) was added dropwise to a cold solution (8-10°C) of crude benzo[1,2,5]thiadiazol-5-yl-acetic acid (Example 9(b), 2.8 g, 14 mmol) in 20 ml of anhydrous THF. Stirring was continued for 1.25 hours while maintaining the temperature below 30°C. The mixture was cooled to 10°C then methanol was added (1.4 ml) and the mixture was left 15 hours in a fridge. The mixture was diluted with ethyl acetate, washed with water then with a saturated aqueous solution of sodium hydrogen carbonate and again with water. The organic phase was separated, dried over magnesium sulphate and evaporated. The residue was triturated in pentane, collected by filtration and dried to give crude) 2-benzo[1,2,5]thiadiazol-5-yl-ethanol (1.8 g, 71%).
δH(CDCl3, 200MHz): 7.94(1H, d), 7.84(1H, d), 7.50(1H, dd), 3.99(2H, t), 3.06(2H, t), 1.58(1H, br).

### (d) Methanesulfonic acid 2-benzo[1,2,5]thiadiazol-5-yl-ethyl ester.

Methanesulfonyl chloride (450 ul, 5.8 mmol) was added dropwise to an ice cooled solution of 2-benzo[1,2,5]thiadiazol-5-yl-ethanol (Example 9(c), 1 g, 5.5 mmol) and triethylamine (840 ul, 6 mmol) in 20 ml of methylene chloride stabilised over amylene. The reaction mixture was allowed to warm to room temperature and stirred for 16 hours. The organic solution was washed with water, dried over magnesium sulphate and evaporated to dryness. The oily residue was triturated 15 hours with diisopropyl ether. The solid was collected by filtration and dried to give methanesulfonic acid 2-benzo[1,2,5]thiadiazol-5-yl-ethyl ester (1.2 g, 78%) which was used without further purification in the next step.
δH(CDCl3, 200MHz): 7.98(1H, d), 7.87(1H, d), 7.50(1H, dd), 4.54(2H, t), 3.26(2H, t), 2.95(3H, s).

### (e) Title compound.

A mixture ofpiperazine-1-carboxylic acid (6-methoxy-[1,5]naphthyridin-4-yl)-amide (Example 5(d) 0.2 g, 0.7 mmol), methanesulfonic acid 2-benzo[1,2,5]thiadiazol-5-yl-ethyl ester (Example 9(d), 0.2 g, 0.77 mmol), triethylamine (110 ul, 0.77 mmol) and acetonitrile (12 ml) was heated under reflux for 16 hours. The mixture was diluted with water and extracted 4 times with methylene chloride. The organic phases was dried over magnesium sulphate and concentrated to dryness. The residue was chromatographed on silica gel eluting with methylene chloride-methanol (97-3). The desired fractions were pooled and concentrated. The residue was crystallised by trituration in diethyl ether. The solid was collected by filtration and dried to give the title compound (0.072 g, 23%) as yellow crystals. mp : 188-190°C.
δH(d6-DMSO,200MHz) : 9.10(1H, br), 8.61(1H, d), 8.31-8.16(2H, m), 8.07-7.89(2H, m), 7.68(1H, dd), 7.29(1H,d), 4.08(3H, s), 3.56(4H, m), 3.01(2H, t), 2.71(2H, t), 2.58(4H, m).

### Example 10 4-(2-Oxo-2-quinoxalin-2-yl-ethyl)-piperazine-1-carboxylic acid (6-methoxy-[1,5]naphthyridin-4-yl)-amide.

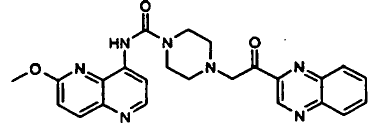

A mixture of piperazine-1-carboxylic acid (6-methoxy-[1,5]naphthyridin-4-yl)-amide (Example 5(d) 400 mg, 1.39 mmol), 2-bromo-1-quinoxalin-2-yl-ethanone [RN 35970-57-1] (385 mg, 1.53 mmol), diisopropyl-ethylamine (359 mg, 2.78 mmol) and THF (30 ml) was stirred at room temperature for 4 hours. The solvent was concentrated, the residue was dissolved in methylene chloride and the organic phase was washed with water, dried over magnesium sulphate and concentrated to dryness. The residue was chromatographed on silica gel eluting with ethyl acetate-methylene chloride (1-1). The compound was then crystallised by trituration in diethyl ether-ethyl acetate (9-1). The solid was collected by filtration and dried to give the title compound (250 mg, 39%) as yellow crystals. mp : 167-169°C.
δH(d-6 DMSO, 200MHz): 9.38(1H, s), 9.12(1H, br), 8.62(1H, d), 8.32=8.14(4H, m), 8.11-7.91(2H, m), 7.30(1H, d), 4.34(2H, s), 4.01(3H, s), 3.65(4H, m), 2.74(4H, m).

### Example 11 4-(2-Hydroxy-2-quinoxalin-2-yl-ethyl)-piperazine-1-carboxylic acid (6-methoxy-[1,5]naphthyridin-4-yl)-amide.

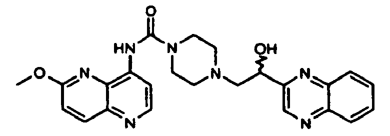

Sodium borohydride (20 mg, 0.526 mmol) was added to a solution of 4-(2-oxo-2-quinoxalin-2-yl-ethyl)-piperazine-1-carboxylic acid (6-methoxy-[1,5]naphthyridin-4-yl)-amide (Example 10, 200 mg, 0.437 mmol) in methanol (5 ml) and the mixture was stirred at room temperature for 2 hours. The mixture was carefully diluted with water and extracted twice with ethyl acetate (2x50 ml). The organic phase was washed with water, dried over magnesium sulphate and concentrated to dryness to give the title compound (100 mg, 49%). mp : 133-135°C.
δH(CDCl3, 200MHz): 9.12(1H, s), 9.06(1H, br), 8.66(1H, d), 8.31(1H, d), 8.22(1H, d), 8.00-8.20(2H, m), 7.72-7.89(2H, m), 7.15(1H, d), 5.14(1H, dd), 4.34(1H, br), 4.07(3H, s), 3.88-3.58(4H,m), 3.08-2.62(6H, m).

### Example 12 4-[2-Oxo-2-(3-oxo-3,4-dihydro-2H-benzo[1,4]oxazin-6-yl)-ethyl]-piperazine-1-carboxylic acid (6-methoxy-[1,5]naphthyridin4-yl)-amide.

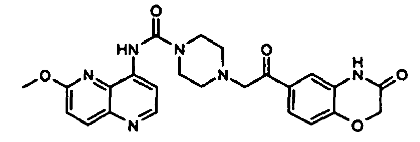

A mixture of piperazine-1-carboxylic acid (6-methoxy-[1,5]naphthyridin-4-yl)-amide (Example 5(d) 450 mg, 1.56 mmol), 6-(2-chloro-ethanoyl)-4H-benzo[1,4]oxazin-3-one [26518-76-3] (345 mg, 1.53 mmol), diisopropyl-ethylamine (DIEA) (359 mg, 2.78 mmol) and THF (25 ml) was stirred at room temperature for 16 hours. The reaction being not finished, the solvent was concentrated, the residue was dissolved in acetonitrile, 200 mg of DIEA were added and the mixture was heated under reflux for 5 hours. After cooling the solid formed was collected by filtration, washed 3 times with a small amount of acetonitrile then 2 times with water and dried to give the title compound as off-white crystals (650 mg, 87%). mp : 274-276.
δH(d6-DMSO,200MHz) 10.88(1H, br), 9.08(1H, s), 8.60(1H, d), 8.31-8.12(2H, m), 7.67(1H, dd), 7.57(1H, d), 7.28(1H, d), 7.04(1H, d), 4.69(2H,s), 4.07(3H,s), 3.82(2H, s), 3.58(4H, m), 2.61(4H, m).

### Example 13 4-[2-Hydroxy-2-(3-oxo-3,4-dihydro-2H-benzo[1,4]oxazin-6-yl)-ethyl]-piperazine-1-carboxylic acid (6-methoxy-[1,5]naphthyridin-4-yl)-amide.

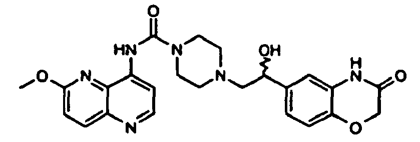

Sodium borohydride (24 mg, 0.63 mmol) was added to a suspension of 4-[2-oxo-2-(3-oxo-3,4-dihydro-2H-benzo[1,4]oxazin-6-yl)-ethyl]-piperazine-1-carboxylic acid (6-methoxy-[1,5]naphthyridin-4-yl)-amide (Example 12, 250 mg, 0.52 mmol) in methanol (10 ml) and the mixture was heated to reflux for 3 hours. This process, addition of 25 mg of sodium borohydride followed by 3 hours of reflux was repeated four times. The solid (unreacted starting material) was filtered off and the solution was concentrated to dryness. The residue was chromatographed on silica gel eluting with ethyl acetate-methanol (first 99-1, then 98-2 finally 97-3) to give the title compound (55 mg, 22%) as white crystals. mp : 231-233°C.
SH(CDCl3, 200MHz) : 9.06(1H, s), 8.66(1H, d), 8.32(1H, d), 8.23(1H, d), 7.90(1H, s), 7.15(1H, d), 7.00-6.82(3H, m), 4.76(1H, dd), 4.61(2H, s), 4.08(3H, s), 3.73(4H, m), 3.00-2.80(2H, m), 2.72-2.48(4H, m), 1.67(1H, br).

The following compounds were prepared by procedures analogous to those described herein:

| | | | | |
|---|---|---|---|---|
| 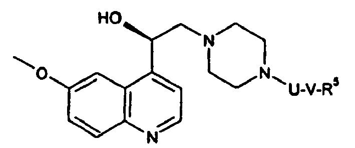 | | | | |

| Example | | Salt | -U-V- | R⁵ |
|---|---|---|---|---|
| 14 | | B | -CH₂-CH₂- | 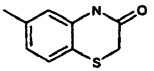 |
| 15 | | R | -CH₂-CH(OH)- | 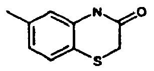 |
| 16 | | R | -CH₂-CH(OH)- | 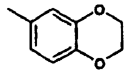 |
| 17 | | S | -CO-CH2- | 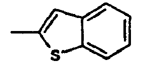 |

| | | | | |
|---|---|---|---|---|
| Salts: B = dihydrochloride R = trihydrochloride S = mesylate | | | | |

### Biological Activity

The MIC (µg/ml) of test compounds against various organisms was determined:
**S. aureus Oxford, S. aureus WCUH29, S. pneumoniae 1629, S. pneumoniae N1387, S. pneumoniae ERY 2, H. influenzae Q1, E. faecalis 7.**
Examples 1-9, 12 and 16 have an MIC of less than or equal to 0.25µg/ml against one or more of the above range of gram positive and gram negative bacteria.
Examples 10, 14 and 15 have an MIC of less than or equal to 2µg/ml against one or more of the above range of gram positive and gram negative bacteria.

## Claims

1. A compound of formula (I) or a pharmaceutically acceptable salt and/or N-oxide thereof: 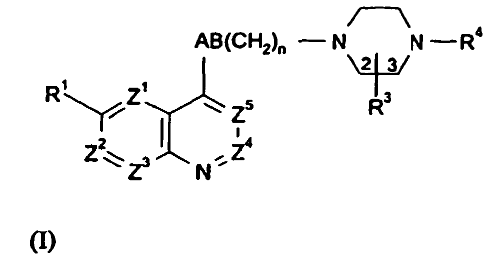 wherein:
one of Z¹, Z², Z³, Z⁴ and Z⁵ is N, one is CR^{1a} and the remainder are CH, or one of Z¹, Z², Z³, Z⁴ and Z⁵ is CR^{1a} and the remainder are CH;
R¹ and R^{1a} are independently selected from hydrogen; hydroxy; (C₁₋₆) alkoxy optionally substituted by (C₁₋₆)alkoxy, amino, piperidyl, guanidino or amidino any of which is optionally N-substituted by one or two (C₁₋₆)alkyl, acyl or (C₁₋₆) alkylsulphonyl groups, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy-substituted (C₁₋₆) alkyl; halogen; (C₁₋₆)alkyl; (C₁₋₆)alkylthio; trifluoromethyl; nitro; azido; acyl; acyloxy; acylthio; (C₁₋₆)alkylsulphonyl; (C₁₋₆)alkylsulphoxide; arylsulphonyl; arylsulphoxide or an amino, piperidyl, guanidino or amidino group optionally N-substituted by one or two (C₁₋₆)alkyl, acyl or (C₁₋₆)alkylsulphonyl groups,
or when Z⁵ is CR^{1a}, R^{1a} may instead be cyano, hydroxymethyl or carboxy, provided that when none of Z¹, Z², Z³, Z⁴ and Z⁵ is N, then R¹ is not hydrogen;
R³ is hydrogen; or
R³ is in the 2- or 3-position and is:
carboxy; (C₁₋₆)alkoxycarbonyl; aminocarbonyl wherein the amino group is optionally substituted by hydroxy, (C₁₋₆)alkyl, hydroxy(C₁₋₆)alkyl, aminocarbonyl(C₁₋₆)alkyl, (C₂₋₆)alkenyl, (C₁₋₆)alkylsulphonyl, trifluoromethylsulphonyl, (C₂₋₆)alkenylsulphonyl, (C₁₋₆)alkoxycarbonyl, (C₁₋₆)alkylcarbonyl, (C₂-₆)alkenyloxycarbonyl or (C₂₋₆) alkenylcarbonyl and optionally further substituted by (C₁₋₆)alkyl, hydroxy(C₁₋₆)alkyl, aminocarbonyl(C₁₋₆)alkyl or (C₂₋₆)alkenyl; cyano; tetrazolyl; 2-oxo-oxazolidinyl optionally substituted by R¹⁰; 3-hydroxy-3-cyclobutene-1,2-dione-4-yl; 2,4-thiazolidinedione-5-yl; tetrazol-5-ylaminocarbonyl; 1,2,4-triazol-5-yl optionally substituted by R¹⁰; or 5-oxo-1,2,4-oxadiazol-3-yl; or
(C₁₋₄)alkyl or ethenyl optionally substituted with any of the groups listed above for R³ and/or 0 to 2 groups R¹² independently selected from:
halogen; (C₁₋₆)alkylthio; trifluoromethyl; (C₁₋₆)alkoxycarbonyl; (C₁₋₆) alkylcarbonyl; (C₂₋₆)alkenyloxycarbonyl; (C₂₋₆)alkenylcarbonyl; hydroxy optionally substituted by (C₁₋₆)alkyl, (C₂₋₆)alkenyl, (C₁₋₆)alkoxycarbonyl, (C₁₋₆)alkylcarbonyl, (C₂₋₆)alkenyloxycarbonyl, (C₂₋₆)alkenylcarbonyl or aminocarbonyl wherein the amino group is optionally substituted by (C₁₋₆)alkyl, (C₂₋₆)alkenyl, (C₁₋₆)alkylcarbonyl or (C₂₋₆)alkenylcarbonyl; amino optionally mono- or disubstituted by (C₁₋₆) alkoxycarbonyl, (C₁₋₆)alkylcarbonyl, (C₂₋₆)alkenyloxycarbonyl, (C₂₋₆) alkenylcarbonyl, (C₁₋₆)alkyl, (C₂₋₆)alkenyl, (C₁₋₆)alkylsulphonyl, (C₂₋₆) alkenylsulphonyl or aminocarbonyl wherein the amino group is optionally substituted by (C₁₋₆)alkyl or (C₂₋₆)alkenyl; aminocarbonyl wherein the amino group is optionally substituted by (C₁₋₆)alkyl, hydroxy(C₁₋₆)alkyl, aminocarbonyl(C₁₋₆)alkyl, (C₂₋₆) alkenyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆) alkyl, aminocarbonyl(C₁₋₆)alkyl or (C₂₋₆)alkenyl; oxo; (C₁₋₆)alkylsulphonyl; (C₂₋₆) alkenylsulphonyl; or (C₁₋₆)aminosulphonyl wherein the amino group is optionally substituted by (C₁₋₆)alkyl or (C₂₋₆)alkenyl;
in addition when R³ is disubstituted with a hydroxy or amino containing substituent and a carboxy containing substituent these may optionally together form a cyclic ester or amide linkage, respectively;
R¹⁰ is selected from (C₁₋₄)alkyl; (C₂₋₄)alkenyl and aryl any of which may be optionally substituted by a group R¹² as defined above; carboxy; and aminocarbonyl wherein the amino group is optionally substituted by hydroxy, (C₁₋₆)alkyl, (C₂₋₆)alkenyl, (C₁₋₆) alkylsulphonyl, trifluoromethylsulphonyl, (C₂₋₆)alkenylsulphonyl, (C₁₋₆) alkoxycarbonyl, (C₁₋₆)alkylcarbonyl, (C₂₋₆)alkenyloxycarbonyl or (C₂₋₆) alkenylcarbonyl;
R⁴ is a group -U-V-R⁵ where R⁵ is an optionally substituted bicyclic carbocyclic or heterocyclic ring system (A): 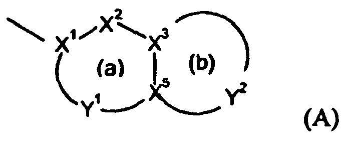 containing up to four heteroatoms in each ring in which
at least one of rings (a) and (b) is aromatic;
X¹ is C or N when part of an aromatic ring or CR¹⁴ or N when part of a non-aromatic ring;
X² is N, NR¹³, O, S(O)ₓ, CO or CR¹⁴ when part of an aromatic or non-aromatic ring or may in addition be CR¹⁴R¹⁵ when part of a non-aromatic ring;
X³ and X⁵ are independently N or C;
Y¹ is a 0 to 4 atom linker group each atom of which is independently selected from N, NR¹³, O, S(O)ₓ, CO and CR¹⁴ when part of an aromatic or non-aromatic ring or may additionally be CR¹⁴R¹⁵ when part of a non-aromatic ring,
Y² is a 2 to 6 atom linker group, each atom of Y² being independently selected from N, NR¹³, O, S(O)ₓ, CO and CR¹⁴ when part of an aromatic or non-aromatic ring or may additionally be CR¹⁴R¹⁵ when part of a non-aromatic ring; each of R¹⁴ and R¹⁵ is independently selected from: H; (C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄) alkenylcarbonyl; (C₁₋₄)alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted as for corresponding substituents in R³; (C₁₋₄) alkylsulphonyl; (C₂₋₄)alkenylsulphonyl; or aminosulphonyl wherein the amino group is optionally substituted by (C₁₋₄)alkyl or (C₂₋₄)alkenyl; aryl; aryl(C₁₋₄)alkyl; and aryl(C₁₋₄) alkoxy;
each R¹³ is independently H; trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; aryl; aryl (C₁₋₄)alkyl; (C₁₋₄)alkoxycarbonyl; (C₁₋₄)alkylcarbonyl; formyl; (C₁₋₆) alkylsulphonyl; or aminocarbonyl wherein the amino group is optionally substituted by (C₁₋₄)alkoxycarbonyl, (C₁₋₄)alkylcarbonyl, (C₂₋₄)alkenyloxycarbonyl, (C₂₋₄) alkenylcarbonyl, (C₁₋₄)alkyl or (C₂₋₄)alkenyl and optionally further substituted by (C₁₋₄)alkyl or (C₂₋₄)alkenyl;
U is selected from CO, SO₂ and CH₂ and V is CR¹⁷ R¹⁸ or U is CH₂ and V is CO or SO₂;
R¹⁷ and R¹⁸ are independently selected from hydrogen; hydroxy optionally substituted by (C₁₋₆)alkyl, (C₂₋₆)alkenyl, (C₁₋₆)alkoxycarbonyl, (C₁₋₆)alkylcarbonyl, (C₂₋₆) alkenyloxycarbonyl, (C₂₋₆)alkenylcarbonyl or aminocarbonyl wherein the amino group is optionally substituted by (C₁₋₆)alky), (C₂₋₆)alkenyl, (C₁₋₆)alkylcarbonyl or (C₂₋₆) alkenylcarbonyl; and amino optionally mono- or disubstituted by (C₁₋₆) alkoxycarbonyl, (C₁₋₆)alkylcarbonyl, (C₂₋₆)alkenyloxycarbonyl, (C₂₋₆) alkenylcarbonyl, (C₁₋₆)alkyl, (C₂₋₆)alkenyl, (C₁₋₆)alkylsulphonyl, (C₂₋₆) alkenylsulphonyl or aminocarbonyl wherein the amino group is optionally substituted by (C₁₋₆)alkyl or (C₂₋₆)alkenyl;
n is 0 and AB is NR¹¹CO, CO-CR⁸R⁹, CR⁶R⁷-CO, NR¹¹SO₂, CR⁶R⁷-SO₂ or CR⁶R⁷⁻CR⁸R⁹, provided that R⁸ and R⁹ are not optionally substituted hydroxy or amino and R⁶ and R⁸ do not represent a bond:
or n is 1 and AB is NR¹¹CO, CO-CR⁸R⁹, CR⁶R⁷-CO, NR¹¹SO₂, CONR¹¹, CR⁶R⁷⁻CR⁸R⁹, O-CR⁸R⁹ or NR¹¹-CR⁸R⁹;
and wherein:
each of R⁶, R⁷, R⁸ and R⁹ is independently selected from: H; (C₁₋₆)alkoxy; (C₁₋₆) alkylthio; halo; trifluoromethyl; azido; (C₁₋₆)alkyl; (C₂₋₆)alkenyl; (C₁₋₆) alkoxycarbonyl; (C₁₋₆)alkylcarbonyl; (C₂₋₆)alkenyloxycarbonyl; (C₂₋₆) alkenylcarbonyl; hydroxy, amino or aminocarbonyl optionally substituted as for corresponding substituents in R³; (C₁₋₆)alkylsulphonyl; (C₂₋₆)alkenylsulphonyl; or (C₁₋₆) aminosulphonyl wherein the amino group is optionally substituted by (C₁₋₆)alkyl or (C₂₋₆)alkenyl;
or R⁶ and R⁸ together represent a bond and R⁷ and R⁹ are as above defmed;
and each R¹¹ is independently H; trifluoromethyl; (C₁₋₆)alkyl; (C₂₋₆)alkenyl; (C₁₋₆) alkoxycarbonyl; (C₁₋₆)alkylcarbonyl; or aminocarbonyl wherein the amino group is optionally substituted by (C₁₋₆)alkoxycarbonyl, (C₁₋₆)alkylcarbonyl, (C₂₋₆) alkenyloxycarbonyl, (C₂₋₆)alkenylcarbonyl, (C₁₋₆)alkyl or (C₂₋₆)alkenyl and optionally further substituted by (C₁₋₆)alkyl or (C₂₋₆)alkenyl;
or where one of R³ and R⁶, R⁷, R⁸ or R⁹ contains a carboxy group and the other contains a hydroxy or amino group they may together form a cyclic ester or amide linkage;
wherein
'heterocyclic' is an aromatic or non-aromatic, single or fused, ring containing up to four hetero-atoms in each ring selected from oxygen, nitrogen and sulphur, and having from 4 to 7 ring atoms, which rings may be unsubstituted or C-substituted by, for example, up to three groups selected from (C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄)alkoxycarbonyl; formyl; (C₁₋₄) alkylcarbonyl; (C₂₋₄)alkenyloxycarbonyl; (C₂₋₄)alkenylcarbonyl; (C₁₋₄) alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted as for corresponding substituents in R³; (C₁₋₄)alkylsulphonyl; (C₂₋₄) alkenylsulphonyl; or aminosulphonyl wherein the amino group is optionally substituted by (C₁₋₄)alkyl or (C₂₋₄)alkenyl; optionally substituted aryl, aryl(C₁₋₄)alkyl or aryl(C₁₋₄) alkoxy and oxo, and wherein any amino group forming part of a single or fused non-aromatic heterocyclic ring as defined above is optionally substituted by trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; aryl; aryl (C₁₋₄)alkyl; (C₁₋₄)alkoxycarbonyl; (C₁₋₄) alkylcarbonyl; formyl; (C₁₋₆)alkylsulphonyl; or aminocarbonyl wherein the amino group is optionally substituted by (C₁₋₄)alkoxycarbonyl, (C₁₋₄)alkylcarbonyl, (C₂₋₄) alkenyloxycarbonyl, (C₂₋₄)alkenylcarbonyl, (C₁₋₄)alkyl or (C₂₋₄)alkenyl and optionally further substituted by (C₁₋₄)alkyl or (C₂₋₄)alkenyl;
'aryl' is phenyl or naphthyl optionally substituted with up to five groups selected from (C₁₋₄)alkylthio; halo; carboxy(C₁-₄)alkyl; halo(C₁₋₄)alkoxy; halo(C₁₋₄)alkyl; (C₁₋₄) alkyl; (C₂₋₄)alkenyl; (C₁₋₄)alkoxycarbonyl; formyl; (C₁₋₄)alkylcarbonyl; (C₂₋₄) alkenyloxycarbonyl; (C₂₋₄)alkenylcarbonyl; (C₁₋₄)alkylcarbonyloxy; (C₁₋4)alkoxycarbonyl(C₁₋₄)alkyl; hydroxy; hydroxy(C₁₋₄)alkyl; mercapto(C₁₋₄)alkyl; (C₁₋₄) alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄) alkenyl; phenyl, phenyl(C₁₋₄)alkyl and phenyl(C₁₋₄)alkoxy;
'acyl' is (C₁₋₆)alkoxycarbonyl, formyl or (C₁₋₆) alkylcarbonyl.

2. A compound according to claim 1 wherein Z⁵ is CH or N, Z³ is CH or CF and Z¹, Z² and Z⁴ are each CH, or Z¹ is N, Z³ is CH or CF and Z², Z⁴ and Z⁵ are each CH.

3. A compound according to any preceding claim wherein R¹ is methoxy and R^{1a} is H or when Z³ is CR^{1a} it may be C-F.

4. A compound according to any preceding claim wherein R³ is hydrogen; CONH₂; 1-hydroxyalkyl; CH₂CO₂H; CH₂CONH₂; -CONHCH₂CONH₂; 1,2-dihydroxyalkyl; CH₂CN; 2-oxo-oxazolidin-5-yl or 2-oxo-oxazolidin-5-yl(C₁₋₄alkyl).

5. A compound according to any preceding claim wherein n is 0 and either A is CHOH and B is CH₂ or A is NH and B is CO.

6. A compound according to any preceding claim wherein -U-V- is -(CH₂)₂-.

7. A compound according to any preceding claim wherein R⁵ is an aromatic heterocyclic ring (A) having 8-11 ring atoms including 2-4 heteroatoms of which at least one is N or NR¹³ or the heterocyclic ring (A) has ring (a) aromatic and ring (b) non-aromatic and Y² has 3-5 atoms including NR¹³, O or S bonded to X⁵ and NHCO bonded via N to X³, or O or NH bonded to X³.

8. A compound according to claim 1 selected from:
2-(2-{4-[(*R*)-2-Hydroxy-2-(6-methoxy-quinolin-4-yl)-ethyl]-piperazin-1-yl}-ethyl)-isoindole-1,3-dione
(R)-2-{4-[2-(4-Fluoro-1H-benzoimidazol-2-yl)-ethyl]-piperazin-1-yl}-1-(6-methoxy-quinolin-4-yl)-ethanol
4-[2-(4-Fluoro-1H-benzoimidazol-2-yl)-ethyl]-piperazine-1-carboxylic acid (6-methoxy-[1,5]naphthyridin-4-yl)-amide
(R)-2- {4-[2-(5-Ftuoro-1H-benzoimidazol-2-yl)-ethyl]-piperazin-1-yl}-1-(6-methoxy-quinolin-4-yl)-ethanol
4-[2-(2,3-Dihydro-benzo[1,4]dioxin-6-yl)-ethyl]-piperazine-1-carboxylic acid (6-methoxy-[1,5]naphthyridin-4-yl)-amide
4-(2-Benzo[1,3]dioxol-5-yl-ethyl)-piperazine-1-carboxylic acid (6-methoxy-[1,5]naphthyridin-4-yl)-amide
4-[2-(3-Oxo-3,4-dihydro-2H-benzo[1,4]thiazin-6-yl)-ethyl]-piperazine-1-carboxylic acid (6-methoxy-[1,5]naphthyridin-4-yl)-amide
4-(2-Quinoxalin-2-yl-ethyl)-piperazine-1-carboxylic acid (b-methoxy-[1,5]naphthyridin-4-yl)-amide
4-(2-Benzo[1,2,5]thiadiazol-5-yl-ethyl)-piperazine-1-carboxylic acid (6-methoxy-[1,5]naphthyridin-4-yl)-amide
4-(2-Oxo-2-quinoxalin-2-yl-ethyl)-piperazine-1-carboxylic acid (6-methoxy-[1,5]naphthyridin-4-yl)-amide
4-(2-Hydroxy-2-quinoxalin-2-yl-ethyl)-piperazine-1-carboxylic acid (6-methoxy-[1,5]naphthyridin-4-yl)-amide
4-[2-Oxo-2-(3-oxo-3,4-dihydro-2H-benzo[1,4]oxazin-b-yl)-ethyl]-piperazine-1-carboxylic acid (6-methoxy-[1,5]naphthyridin-4-yl)-amide and
4-[2-Hydroxy-2-(3-oxo-3,4-dihydro-2H-benzo[1,4]oxazin-6-yl)-ethyl]-piperazine-1-carboxylic acid (6-methoxy-[1,5]naphthyridin-4-yl)-amide
or a pharmaceutically acceptable salt and/or N-oxide thereof.

9. The use of a compound according to claim 1, in the manufacture of a medicament for use in the treatment of bacterial infections in mammals.

10. A pharmaceutical composition comprising a compound according to claim 1, and a pharmaceutically acceptable carrier.

11. A process for preparing a compound according to claim 1, which process comprises reacting a compound of formula (IV) with a compound of formula (V): 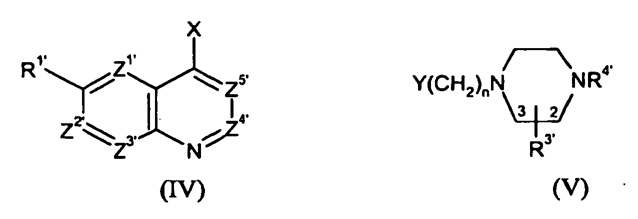 wherein n is as defined in formula (I); Z^{1'}, Z^{2'}, Z^{3'}, Z^{4'}, Z^{5'}, R^{1'}, R^{3'} and R^{4'} are Z¹, Z², Z³, Z⁴, Z⁵, R¹, R³ and R⁴ as defined in formula (I) or groups convertible thereto;
and X and Y may be the following combinations:
(i) X is A'-COW, Y is H and n is 0;
(ii) X is CR⁶=CR⁸R⁹, Y is H and n is 0;
(iii) X is oxirane, Y is H and n is 0;
(iv) X is N=C=O and Y is H and n is 0;
(v) one of X and Y is CO₂R^{y} and the other is CH₂CO₂R^{x};
(vi) X is CHR⁶R⁷ and Y is C(=O)R⁸;
(vii) X is CR⁷=PR^{z}₃ and Y is C(=O)R⁹ and n=1;
(viii) X is C(=O)R⁷ and Y is CR⁹=PR^{z}₃ and n=1;
(ix) Y is COW and X is NHR^{11'} or NR11'COW and n=0 or 1 or when n=1 X is COW and Y is NHR^{11'} or NR11'COW;
(x) X is NHR^{11'} and Y is C(=O)R⁸ and n=1;
(xi) X is NHR^{11'} and Y is CR⁸R⁹W and n=1;
(xii) X is NR^{11'} COCH₂W or NR¹¹'SO₂CH₂W and Y is H and n=0;
(xiii) X is CR⁶R⁷SO₂W and Y is H and n=0;
(xiv) X is W or OH and Y is CH₂OH and n is 1;
(xv) X is NHR^{11'} and Y is SO₂W or X is NR^{11'}SO₂W and Y is H, and n is 0;
in which W is a leaving group, e.g. halo or imidazolyl; R^{x} and R^{y} are (C₁₋₆)alkyl; R^{z} is aryl or (C₁₋₆₎alkyl; A' and NR^{11'} are A and NR¹¹ as defined in formula (I), or groups convertible thereto; and oxirane is: 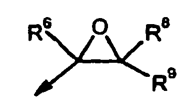 wherein R⁶, R⁸ and R⁹ are as defined in formula (I);
and thereafter optionally or as necessary converting A', Z^{1'}, Z^{2'}, Z^{3'}, Z^{4'}, Z^{5'}, R^{1'}, R^{3'}, R^{4'} and NR^{11'} to A, Z¹, Z², Z³, Z⁴, Z⁵, R¹, R³, R⁴ and NR¹¹; converting A-B to other A-B, interconverting R¹, R³ and/or R⁴, and/or forming a pharmaceutically acceptable salt and/or N-oxide thereof.

## Patentansprüche

1. Verbindung der Formel (I) oder ein pharmazeutisch verträgliches Salz und/oder N-Oxid davon: 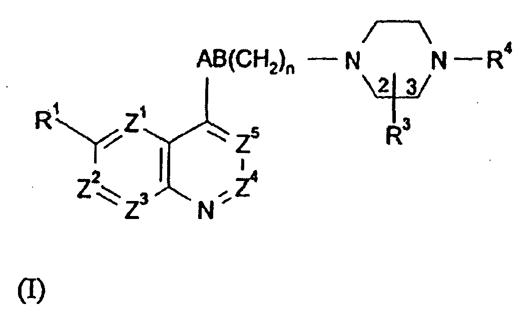 wobei:
eines von Z¹, Z², Z³, Z⁴ und Z⁵ N ist, eines CR^{1a} ist und die übrigen CH sind, oder eines von Z¹, Z², Z³, Z⁴ und Z⁵ CR^{1a} ist und die übrigen CH sind;
R¹ und R^{1a} unabhängig voneinander ausgewählt sind aus Wasserstoff; Hydroxy; (C₁₋₆)Alkoxy, das gegebenenfalls mit (C₁₋₆)Alkoxy, Amino, Piperidyl, Guanidino oder Amidino, von denen jedes gegebenenfalls mit einem oder zwei (C₁₋₆)Alkyl-, Acyl- oder (C₁₋₆)Alkylsulfonyl-Resten N-substituiert ist, (C₁₋₆)Alkylthio, Heterocyclylthio, Heterocyclyloxy, Arylthio, Aryloxy, Acylthio, Acyloxy oder (C₁₋₆)Alkylsulfonyloxy substituiert ist; (C₁₋₆)Alkoxy-substituiertem (C₁₋₆)Alkyl; Halogen; (C₁₋₆)Alkyl; (C₁₋₆)Alkylthio; Trifluormethyl; Nitro; Azido; Acyl; Acyloxy; Acylthio; (C₁₋₆)Alkylsulfonyl; (C₁₋₆)Alkylsulfoxid; Arylsulfonyl; Arylsulfoxid oder einer Amino-, Piperidyl-, Guanidino- oder Amidino-Gruppe, die gegebenenfalls mit einem oder zwei (C₁₋₆)Alkyl-, Acyl- oder (C₁₋₆)Alkylsulfonyl-Resten N-substituiert ist,
oder wenn Z⁵ CR^{1a} ist, R^{1a} stattdessen Cyano, Hydroxymethyl oder Carboxy sein kann,
mit der Maßgabe, dass, wenn keines von Z¹, Z², Z³, Z⁴ und Z⁵ N ist, R¹ nicht Wasserstoff ist;
R³ Wasserstoff ist; oder
R³ in der 2- oder 3-Position ist und:
Carboxy; (C₁₋₆)Alkoxycarbonyl; Aminocarbonyl, wobei die Aminogruppe gegebenenfalls mit Hydroxy, (C₁₋₆)Alkyl, Hydroxy(C₁₋₆)alkyl, Aminocarbonyl(C₁₋₆)alkyl, (C₂₋₆)Alkenyl, (C₁₋₆)Alkylsulfonyl, Trifluormethylsulfonyl, (C₂₋₆)Alkenylsulfonyl, (C₁₋₆)Alkoxycarbonyl, (C₁₋₆)Alkylcarbonyl, (C₂₋₆)Alkenyloxycarbonyl oder (C₂₋₆)Alkenylcarbonyl substituiert ist und gegebenenfalls ferner mit (C₁₋₆)Alkyl, Hydroxy(C₁₋₆)alkyl, Aminocarbonyl(C₁₋₆)alkyl oder (C₂₋₆)Alkenyl substituiert ist; Cyano; Tetrazolyl; 2-Oxo-oxazolidinyl, das gegebenenfalls mit R¹⁰ substituiert ist; 3-Hydroxy-3-cyclobuten-1,2-dion-4-yl; 2,4-Thiazolidindion-5-yl; Tetrazol-5-ylaminocarbonyl; 1,2,4-Triazol-5-yl, das gegebenenfalls mit R¹⁰ substituiert ist; oder 5-Oxo-1,2,4-oxadiazol-3-yl ist; oder
(C₁₋₄)Alkyl oder Ethenyl ist, gegebenenfalls substituiert mit einem der vorstehend für R³ angeführten Reste und/oder 0 bis 2 Resten R¹², die unabhängig voneinander ausgewählt sind aus:
Halogen; (C₁₋₆)Alkylthio; Trifluormethyl; (C₁₋₆)Alkoxycarbonyl; (C₁₋₆)Alkylcarbonyl; (C₂₋₆)Alkenyloxycarbonyl; (C₂₋₆)Alkenylcarbonyl; Hydroxy, das gegebenenfalls substituiert ist mit (C₁₋₆)Alkyl, (C₂₋₆)Alkenyl, (C₁₋₆)Alkoxycarbonyl, (C₁₋₆)Alkylcarbonyl, (C₂₋₆)Alkenyloxycarbonyl, (C₂₋₆)Alkenylcarbonyl oder Aminocarbonyl, wobei die Aminogruppe gegebenenfalls mit (C₁₋₆)Alkyl, (C₂₋₆)Alkenyl, (C₁₋₆)Alkylcarbonyl oder (C₂₋₆)Alkenylcarbonyl substituiert ist; Amino, das gegebenenfalls mono- oder disubstituiert ist mit (C₁₋₆)Alkoxycarbonyl, (C₁₋₆)Alkylcarbonyl, (C₂₋₆)Alkenyloxycarbonyl, (C₂₋₆)Alkenylcarbonyl, (C₁₋₆)Alkyl, (C₂₋₆)Alkenyl, (C₁₋₆)Alkylsulfonyl, (C₂₋₆)Alkenylsulfonyl oder Aminocarbonyl, wobei die Aminogruppe gegebenenfalls mit (C₁₋₆)Alkyl oder (C₂₋₆)Alkenyl substituiert ist; Aminocarbonyl, wobei die Aminogruppe gegebenenfalls substituiert ist mit (C₁₋₆)Alkyl, Hydroxy(C₁₋₆)alkyl, Aminocarbonyl(C₁₋₆)alkyl, (C₂₋₆)Alkenyl, (C₁₋₆)Alkoxycarbonyl, (C₁₋₆)Alkylcarbonyl, (C₂₋₆)Alkenyloxycarbonyl oder (C₂₋₆)Alkenylcarbonyl und gegebenenfalls ferner substituiert ist mit (C₁₋₆)Alkyl, Hydroxy(C₁₋₆)alkyl, Aminocarbonyl(C₁₋₆)alkyl oder (C₂₋₆)Alkenyl; Oxo; (C₁₋₆)Alkylsulfonyl; (C₂₋₆)Alkenylsulfonyl oder (C₁₋₆)Aminosulfonyl, wobei die Aminogruppe gegebenenfalls mit (C₁₋₆)Alkyl oder (C₂₋₆)Alkenyl substituiert ist;
ferner, wenn R³ mit einem Hydroxy- oder Amino-enthaltenden Substituenten und einem Carboxy-enthaltenden Substituenten disubstituiert ist, diese gegebenenfalls zusammen eine cyclische Ester- bzw. Amidbindung bilden können;
R¹⁰ ausgewählt ist aus (C₁₋₄)Alkyl, (C₂₋₄)Alkenyl und Aryl, von denen jedes gegebenenfalls mit einem wie vorstehend definierten Rest R¹² substituiert sein kann; Carboxy; und Aminocarbonyl, wobei die Aminogruppe gegebenenfalls mit Hydroxy, (C₁₋₆)Alkyl, (C₂₋₆)Alkenyl, (C₁₋₆)Alkylsulfonyl, Trifluormethylsulfonyl, (C₂₋₆)Alkenylsulfonyl, (C₁₋₆)Alkoxycarbonyl, (C₁₋₆)Alkylcarbonyl, (C₂₋₆)Alkenyloxycarbonyl oder (C₂₋₆)Alkenylcarbonyl substituiert ist;
R⁴ ein Rest -U-V-R⁵ ist, wobei R⁵ ein gegebenenfalls substituiertes bicyclisches carbocyclisches oder heterocyclisches Ringsystem (A) ist: 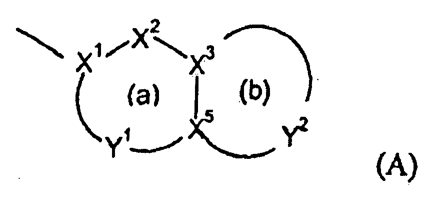 das bis zu vier Heteroatome in jedem Ring enthält, wobei
mindestens einer der Ringe (a) und (b) aromatisch ist;
X¹ C oder N ist, wenn es Teil eines aromatischen Rings ist, oder CR¹⁴ oder N ist, wenn es Teil eines nicht aromatischen Rings ist;
X² N, NR¹³, O, S(O)ₓ, CO oder CR¹⁴ ist, wenn es Teil eines aromatischen oder nicht aromatischen Rings ist, oder ferner CR¹⁴R¹⁵ sein kann, wenn es Teil eines nicht aromatischen Rings ist;
X³ und X⁵ unabhängig voneinander N oder C sind;
Y¹ eine Verbindungsgruppe mit 0 bis 4 Atomen ist, wobei jedes Atom unabhängig aus N, NR¹³, O, S(O)ₓ, CO und CR¹⁴ ausgewählt ist, wenn es Teil eines aromatischen oder nicht aromatischen Rings ist oder ferner CR¹⁴R¹⁵ sein kann, wenn es Teil eines nicht aromatischen Rings ist,
Y² eine Verbindungsgruppe mit 2 bis 6 Atomen ist, wobei jedes Atom von Y² unabhängig aus N, NR¹³, O, S(O)ₓ, CO und CR¹⁴ ausgewählt ist, wenn es Teil eines aromatischen oder nicht aromatischen Rings ist, oder ferner CR¹⁴R¹⁵ sein kann, wenn es Teil eines nicht aromatischen Rings ist;
jedes von R¹⁴ und R¹⁵ unabhängig ausgewählt ist aus H; (C₁₋₄)Alkylthio; Halogen; Carboxy(C₁₋₄)alkyl; Halogen(C₁₋₄)alkoxy; Halogen(C₁₋₄)alkyl; (C₁₋₄)Alkyl; (C₂₋₄)Alkenyl; (C₁₋₄)Alkoxycarbonyl; Formyl; (C₁₋₄)Alkylcarbonyl; (C₂₋₄)Alkenyloxycarbonyl; (C₂₋₄)Alkenylcarbonyl; (C₁₋₄)Alkylcarbonyloxy; (C₁₋₄)Alkoxycarbonyl(C₁₋₄)alkyl; Hydroxy; Hydroxy(C₁₋₄)alkyl; Mercapto(C₁₋₄)alkyl; (C₁₋₄)Alkoxy; Nitro; Cyano; Carboxy; Amino oder Aminocarbonyl, die gegebenenfalls wie die entsprechenden Substituenten bei R³ substituiert sind; (C₁₋₄)Alkylsulfonyl; (C₂₋₄)Alkenylsulfonyl; oder Aminosulfonyl, wobei die Aminogruppe gegebenenfalls mit (C₁₋₄)Alkyl oder (C₂₋₄)Alkenyl substituiert ist; Aryl; Aryl(C₁₋₄)alkyl; und Aryl(C₁₋₄)alkoxy;
jedes R¹³ unabhängig H; Trifluormethyl; (C₁₋₄)Alkyl, das gegebenenfalls mit Hydroxy, (C₁₋₆)Alkoxy, (C₁₋₆)Alkylthio, Halogen oder Trifluormethyl substituiert ist; (C₂₋₄)Alkenyl; Aryl; Aryl(C₁₋₄)alkyl; (C₁₋₄)Alkoxycarbonyl; (C₁₋₄)Alkylcarbonyl; Formyl; (C₁₋₆)Alkylsulfonyl; oder Aminocarbonyl ist, wobei die Aminogruppe gegebenenfalls mit (C₁₋₄)Alkoxycarbonyl, (C₁₋₄)Alkylcarbonyl, (C₂₋₄)Alkenyloxycarbonyl, (C₂₋₄)Alkenylcarbonyl, (C₁₋₄)Alkyl oder (C₂₋₄)Alkenyl substituiert ist und gegebenenfalls ferner mit (C₁₋₄)Alkyl oder (C₂₋₄)Alkenyl substituiert ist;
U aus CO, SO₂ und CH₂ ausgewählt ist und V CR¹⁷R¹⁸ ist oder U CH₂ ist und V CO oder SO₂ ist;
R¹⁷ und R¹⁸ unabhängig ausgewählt sind aus Wasserstoff; Hydroxy, das gegebenenfalls substituiert ist mit (C₁₋₆)Alkyl, (C₂₋₆)Alkenyl, (C₁₋₆)Alkoxycarbonyl, (C₁₋₆)Alkylcarbonyl, (C₂₋₆)Alkenyloxycarbonyl, (C₂₋₆)Alkenylcarbonyl oder Aminocarbonyl, wobei die Aminogruppe gegebenenfalls mit (C₁₋₆)Alkyl, (C₂₋₆)Alkenyl, (C₁₋₆)Alkylcarbonyl oder (C₂₋₆)Alkenylcarbonyl substituiert ist; und Amino, das gegebenenfalls mono- oder disubstituiert ist mit (C₁₋₆)Alkoxycarbonyl, (C₁₋₆)Alkylcarbonyl, (C₂₋₆)Alkenyloxycarbonyl, (C₂₋₆)Alkenylcarbonyl, (C₁₋₆)Alkyl, (C₂₋₆)Alkenyl, (C₁₋₆)Alkylsulfonyl, (C₂₋₆)Alkenylsulfonyl oder Aminocarbonyl, wobei die Aminogruppe gegebenenfalls mit (C₁₋₆)Alkyl oder (C₂₋₆)Alkenyl substituiert ist;
n gleich 0 ist und AB NR¹¹CO, CO-CR⁸R⁹, CR⁶R⁷-CO, NR¹¹SO₂, CR⁶R⁷-SO₂ oder CR⁶R⁷-CR⁸R⁹ ist, mit der Maßgabe, dass R⁸ und R⁹ nicht gegebenenfalls substituiertes Hydroxy oder Amino sind und R⁶ und R⁸ keine Bindung bedeuten;
oder n gleich 1 ist und AB NR¹¹CO, CO-CR⁸R⁹, CR⁶R⁷-CO, NR¹¹SO₂, CONR¹¹, CR⁶R⁷⁻CR⁸R⁹, O-CR⁸R⁹ oder NR¹¹-CR⁸R⁹ ist;
und wobei:
jedes von R⁶, R⁷, R⁸ und R⁹ unabhängig ausgewählt ist aus H; (C₁₋₆)Alkoxy; (C₁₋₆)Alkylthio; Halogen; Trifluormethyl; Azido; (C₁₋₆)Alkyl; (C₂₋₆)Alkenyl; (C₁₋₆)Alkoxycarbonyl; (C₁₋₆)Alkylcarbonyl; (C₂₋₆)Alkenyloxycarbonyl; (C₂₋₆)Alkenylcarbonyl; Hydroxy, Amino oder Aminocarbonyl, die gegebenenfalls wie die entsprechenden Substituenten bei R³ substituiert sind; (C₁₋₆)Alkylsulfonyl; (C₂₋₆)Alkenylsulfonyl; oder (C₁₋₆)Aminosulfonyl, wobei die Aminogruppe gegebenenfalls mit (C₁₋₆)Alkyl oder (C₂₋₆)Alkenyl substituiert ist; oder R⁶ und R⁸ zusammen eine Bindung bedeuten und R⁷ und R⁹ wie vorstehend definiert sind;
und jedes R¹¹ unabhängig H; Trifluormethyl; (C₁₋₆)Alkyl; (C₂₋₆)Alkenyl; (C₁₋₆)Alkoxycarbonyl; (C₁₋₆)Alkylcarbonyl; oder Aminocarbonyl ist, wobei die Aminogruppe gegebenenfalls mit (C₁₋₆)Alkoxycarbonyl, (C₁₋₆)Alkylcarbonyl, (C₂₋₆)Alkenyloxycarbonyl, (C₂₋₆)Alkenylcarbonyl, (C₁₋₆)Alkyl oder (C₂₋₆)Alkenyl substituiert ist und gegebenenfalls ferner mit (C₁₋₆)Alkyl oder (C₂₋₆)Alkenyl substituiert ist;
oder, wenn eines von R³ und R⁶, R⁷, R⁸ oder R⁹ eine Carboxygruppe enthält und das andere eine Hydroxy- oder Aminogruppe enthält, diese zusammen eine cyclische Ester- oder Amidbindung bilden können;
wobei
"heterocyclisch" ein aromatischer oder nicht aromatischer, einzelner oder kondensierter Ring ist, der bis zu 4 Heteroatome, ausgewählt aus Sauerstoff, Stickstoff und Schwefel, in jedem Ring enthält, und 4 bis 7 Ringatome aufweist, wobei die Ringe unsubstituiert oder C-substituiert sein können mit bis zu drei Resten, ausgewählt aus (C₁₋₄)Alkylthio; Halogen, Carboxy(C₁₋₄)alkyl; Halogen(C₁₋₄)alkoxy; Halogen(C₁₋₄)alkyl; (C₁₋₄)Alkyl; (C₂₋₄)Alkenyl; (C₁₋₄)Alkoxycarbonyl; Formyl; (C₁₋₄)Alkylcarbonyl; (C₂₋₄)Alkenyloxycarbonyl; (C₂₋₄)Alkenylcarbonyl; (C₁₋₄)Alkylcarbonyloxy; (C₁₋₄)Alkoxycarbonyl(C₁₋₄)alkyl; Hydroxy; Hydroxy(C₁₋₄)alkyl; Mercapto(C₁₋₄)alkyl; (C₁₋₄)Alkoxy; Nitro; Cyano; Carboxy; Amino oder Aminocarbonyl, die gegebenenfalls wie die entsprechenden Substituenten bei R³ substituiert sind; (C₁₋₄)Alkylsulfonyl; (C₂₋₄)Alkenylsulfonyl; oder Aminosulfonyl, wobei die Aminogruppe gegebenenfalls mit (C₁₋₄)Alkyl oder (C₂₋₄)Alkenyl substituiert ist; gegebenenfalls substituiertem Aryl, Aryl(C₁₋₄)alkyl oder Aryl(C₁₋₄)alkoxy und Oxo und wobei jede Aminogruppe, die Teil eines einzelnen oder kondensierten nicht aromatischen heterocyclischen Rings bildet, der wie vorstehend definiert ist, gegebenenfalls substituiert ist mit Trifluormethyl; (C₁₋₄)Alkyl, das gegebenenfalls mit Hydroxy, (C₁₋₆)Alkoxy, (C₁₋₆)Alkylthio, Halogen oder Trifluormethyl substituiert ist; (C₂₋₄)Alkenyl; Aryl; Aryl(C₁₋₄)alkyl; (C₁₋₄)Alkoxycarbonyl; (C₁₋₄)Alkylcarbonyl; Formyl; (C₁₋₆)Alkylsulfonyl; oder Aminocarbonyl, wobei die Aminogruppe gegebenenfalls mit (C₁₋₄)Alkoxycarbonyl, (C₁₋₄)Alkylcarbonyl, (C₂₋₄)Alkenyloxycarbonyl, (C₂₋₄)Alkenylcarbonyl, (C₁₋₄)Alkyl oder (C₂₋₄)Alkenyl substituiert ist und gegebenenfalls ferner mit (C₁₋₄)Alkyl oder (C₂₋₄)Alkenyl substituiert ist;
"Aryl" Phenyl oder Naphthyl ist, die gegebenenfalls mit bis zu fünf Resten substituiert sind, ausgewählt aus (C₁₋₄)Alkylthio; Halogen, Carboxy(C₁₋₄)alkyl; Halogen(C₁₋₄)alkoxy; Halogen(C₁₋₄)alkyl; (C₁₋₄)Alkyl; (C₂₋₄)Alkenyl; (C₁₋₄)Alkoxycarbonyl; Formyl; (C₁₋₄)Alkylcarbonyl; (C₂₋₄)Alkenyloxycarbonyl; (C₂₋₄)Alkenylcarbonyl; (C₁₋₄)Alkylcarbonyloxy; (C₁₋₄)Alkoxycarbonyl(C₁₋₄)alkyl; Hydroxy; Hydroxy(C₁₋₄)alkyl; Mercapto(C₁₋₄)alkyl; (C₁₋₄)Alkoxy; Nitro; Cyano; Carboxy; Amino oder Aminocarbonyl, die gegebenenfalls wie die vorstehenden Substituenten bei R³ substituiert sind; (C₁₋₄)Alkylsulfonyl; (C₂₋₄)Alkenylsulfonyl; oder Aminosulfonyl, wobei die Aminogruppe gegebenenfalls mit (C₁₋₄)Alkyl oder (C₂₋₄)Alkenyl; Phenyl; Phenyl(C₁₋₄)alkyl und Phenyl(C₁₋₄)alkoxy substituiert ist;
"Acyl" (C₁₋₆)Alkoxycarbonyl, Formyl oder (C₁₋₆)Alkylcarbonyl ist.

2. Verbindung nach Anspruch 1, wobei Z⁵ CH oder N ist, Z³ CH oder CF ist und Z¹, Z² und Z⁴ jeweils CH sind, oder Z¹ N ist, Z³ CH oder CF ist und Z², Z⁴ und Z⁵ jeweils CH sind.

3. Verbindung nach einem der vorstehenden Ansprüche, wobei R¹ Methoxy ist und R^{1a} H ist oder, wenn Z³ CR^{1a} ist, C-F sein kann.

4. Verbindung nach einem der vorstehenden Ansprüche, wobei R³ Wasserstoff, CONH₂, 1-Hydroxyalkyl; CH₂CO₂H; CH₂CONH₂; ―CONHCH₂CONH₂; 1,2-Dihydroxyalkyl; CH₂CN; 2-Oxo-oxazolidin-5-yl oder 2-Oxo-oxazolidin-5-yl(C₁₋₄alkyl) ist.

5. Verbindung nach einem der vorstehenden Ansprüche, wobei n 0 ist und entweder A CHOH ist und B CH₂ ist oder A NH ist und B CO ist.

6. Verbindung nach einem der vorstehenden Ansprüche, wobei -U-V- gleich -(CH₂)₂- ist.

7. Verbindung nach einem der vorstehenden Ansprüche, wobei R⁵ ein aromatischer heterocyclischer Ring (A) mit 8 bis 11 Ringatomen, einschließlich 2 bis 4 Heteroatomen, ist, von denen mindestens eines N oder NR¹³ ist, oder der heterocyclische Ring (A) einen aromatischen Ring (a) und einen nicht aromatischen Ring (b) aufweist und Y² 3 bis 5 Atome, einschließlich N-R¹³, O oder S, an X⁵ gebunden, und NHCO, über N an X³ gebunden, oder O oder NH, an X³ gebunden, aufweist.

8. Verbindung nach Anspruch 1, ausgewählt aus
2-(2- {4-[(R)-2-Hydroxy-2-(6-methoxy-chinolin-4-yl)-ethyl]-piperazin-1-yl}-ethyl)-isoindol-1,3-dion
(R)-2- {4-[2-(4-Fluor-1H-benzimidazol-2-yl)-ethyl]-piperazin-1-yl}-1-(6-methoxy-chinolin-4-yl)-ethanol
4-[2-(4-Fluor-1H-benzimidazol-2-yl)-ethyl]-piperazin-1-carbonsäure-(6-methoxy-[1,5]naphthyridin-4-yl)-amid
(R)-2- {4-[2-(5-Fluor-1H-benzimidazol-2-yl)-ethyl]-piperazin-1-yl}-1-(6-methoxy-chinolin-4-yl)-ethanol
4-[2-(2,3-Dihydro-benzo[1,4]dioxin-6-yl)-ethyl]-piperazin-1-carbonsäure-(6-methoxy-[1,5]naphthyridin-4-yl)-amid
4-(2-Benzo[1,3]dioxol-5-yl-ethyl)-piperazin-1-carbonsäure-(6-methoxy-[1,5]naphthyridin-4-yl)-amid
4-[2-(3-Oxo-3,4-dihydro-2H-benzo[1,4]thiazin-6-yl)-ethyl]-piperazin-1-carbonsäure-(6-methoxy-[1,5]naphthyridin-4-yl)-amid
4-(2-Chinoxalin-2-yl-ethyl)-piperazin-1-carbonsäure-(6-methoxy-[1,5]naphthyridin-4-yl)-amid
4-(2-Benzo[1,2,5]thiadiazol-5-yl-ethyl)-piperazin-1-carbonsäure-(6-methoxy-[1,5]naphthyridin-4-yl)-amid
4-(2-Oxo-2-chinoxalin-2-yl-ethyl)-piperazin-1-carbonsäure-(6-methoxy-[1,5]naphthyridin-4-yl)-amid
4-(2-Hydroxy-2-chinoxalin-2-yl-ethyl)-piperazin-1-carbonsäure-(6-methoxy-[1,5]naphthyridin-4-yl)-amid
4-[2-Oxo-2-(3-oxo-3,4-dihydro-2H-benzo[1,4]oxazin-6-yl)-ethyl]-piperazin-1-carbonsäure-(6-methoxy-[1,5]naphthyridin-4-yl)-amid und
4-[2-Hydroxy-2-(3-oxo-3,4-dihydro-2H-benzo[1,4]oxazin-6-yl)-ethyl]-piperazin-1-carbonsäure-(6-methoxy-[1,5]naphthyridin-4-yl)-amid
oder ein pharmazeutisches verträgliches Derivat davon.

9. Verwendung einer Verbindung nach Anspruch 1 zur Herstellung eines Medikaments zur Verwendung bei der Behandlung von bakteriellen Infektionen bei Säugern.

10. Arzneimittel, umfassend eine Verbindung nach Anspruch 1 und einen pharmazeutisch verträglichen Träger.

11. Verfahren zur Herstellung einer Verbindung nach Anspruch 1, wobei das Verfahren Umsetzen einer Verbindung der Formel (IV) mit einer Verbindung der Formel (V) umfasst: 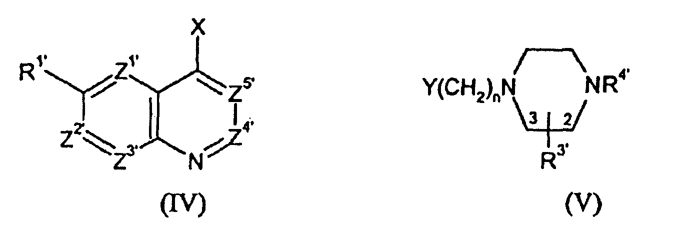 wobei n wie in Formel (I) definiert ist; Z^{1'}, Z^{2'}, Z^{3'}, Z^{4'}, Z^{5'}, R^{1'}, R^{3'} und R^{4'} Z¹, Z², Z³, Z⁴, Z⁵, R¹, R³ und R⁴ wie in Formel (I) definiert oder dazu konvertierbare Reste sind; und X und Y die folgenden Kombinationen sein können:
(i) X ist A'-COW, Y ist H und n ist 0;
(ii) X ist CR⁶=CR⁸R⁹, Y ist H und n ist 0;
(iii) X ist Oxiran; Y ist H und n ist 0;
(iv) X ist N=C=O und Y ist H und n ist 0;
(v) eines von X und Y ist CO₂R^{Y} und das andere ist CH₂CO₂R^{X};
(vi) X ist CHR⁶R⁷ und Y ist C(=O)R⁸;
(vii) X ist CR⁷=PR^{Z}₃ und Y ist C(=O)R⁹ und n=1;
(viii) X ist C(=O)R⁷ und Y ist CR⁹=PR^{Z}₃ und n=1;
(ix) Y ist COW und X ist NHR^{11'} oder NR^{11'}COW und n=0 oder 1 oder wenn n=1, dann ist X COW und Y ist NHR^{11'} oder NR^{11'} COW;
(x) X ist NHR^{11'} und Y ist C(=O)R⁸ und n=1;
(xi) X ist NHR^{11'} und Y ist CR⁸R⁹W und n=1;
(xii) X ist NR^{11'}COCH₂W oder NR^{11'}SO₂CH₂W und Y ist H und n=0;
(xiii) X ist CR⁶R⁷SO₂W und Y ist H und n=0;
(xiv) X ist W oder OH und Y ist CH₂OH und n ist 1;
(xv) X ist NHR^{11'} und Y ist SO₂W oder X ist NR^{11'}SO₂W und Y ist H und n ist 0;
wobei W eine Abgangsgruppe, z.B. Halogen oder Imidazolyl, ist; R^{X} und R^{Y} (C₁₋₆)Alkyl sind; R^{Z} Aryl oder (C₁₋₆)Alkyl ist; A' und NR^{11'} gleich A und NR¹¹ wie in Formel (I) definiert oder dazu konvertierbare Gruppen sind; und Oxiran: 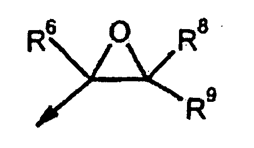 ist, wobei R⁶, R⁸ und R⁹ wie in Formel (I) definiert sind;
und anschließend gegebenenfalls oder bei Bedarf Umwandeln von A', Z^{1'}, Z^{2'}, Z^{3'}, Z^{4'}, Z^{5'}, R^{1'}, R^{3'}, R^{4'} und NR^{11'} in A, Z¹, Z², Z³, Z⁴, Z⁵, R¹, R³, R⁴ und NR¹¹; Umwandeln von A-B in ein anderes A-B; ineinander Umwandeln von R¹, R³ und/oder R⁴, und/oder Bilden eines pharmazeutisch verträglichen Salzes und/oder N-Oxids davon.

## Revendications

1. Composé de formule (I) ou un de ses sels et/ou N-oxydes pharmaceutiquement acceptables : 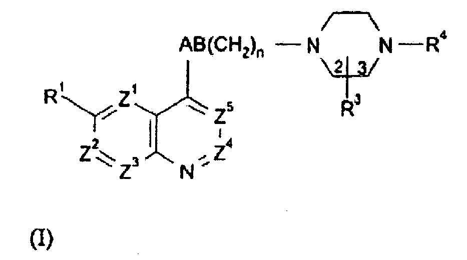 formule dans laquelle :
un des groupes Z¹, Z², Z³, Z⁴ et Z⁵ représente N, un groupe représente un groupe CR^{1a} et le reste représente des groupes CH, ou bien des groupes Z¹, Z², Z³, Z⁴ et Z⁵ représente un groupe CR^{1a} et le reste représente des groupes CH ;
R¹ et R^{1a} sont choisis indépendamment entre un atome d'hydrogène ; des groupes hydroxy ; alkoxy en C₁ à C₆ facultativement substitué avec un substituant alkoxy en C₁ à C₆, amino, pipéridyle, guanidino ou amidino dont n'importe lequel est facultativement N-substitué avec un ou deux groupes alkyle en C₁ à C₆, acyle ou alkylsulfonyle en C₁ à C₆, alkylthio en C₁ à C₆, hétérocyclylthio, hétérocyclyloxy, arylthio, aryloxy, acylthio, acyloxy ou alkylsulfonyloxy en C₁ à C₆ ; alkyle en C₁ à C₆ à substituant alkoxy en C₁ à C₆ ; halogéno ; alkyle en C₁ à C₆ ; alkylthio en C₁ à C₆ ; trifluorométhyle ; nitro; azido ; acyle ; acyloxy ; acylthio ; alkylsulfonyle en C₁ à C₆ ; alkylsulfoxyde en C₁ à C₆ ; arylsulfonyle ; arylsulfoxyde ou un groupe amino, pipéridyle, guanidino ou amidino facultativement N-substitué avec un ou deux groupes alkyle en C₁ à C₆, acyle ou alkylsulfonyle en C₁ à C₆,
ou bien, lorsque Z⁵ représente un groupe CR^{1a}, R^{1a} peut représenter en variante un groupe cyano, hydroxyméthyle ou carboxy,
sous réserve que, lorsqu'aucun des groupes Z¹, Z², Z³, Z⁴ et Z⁵ ne représente N, alors R¹ ne représente pas un atome d'hydrogène ;
R³ représente un atome d'hydrogène ; ou
R³ est en position 2 ou 3 et représente un groupe :
carboxy ; (alkoxy en C₁ à C₆)carbonyle ; aminocarbonyle dans lequel le groupe amino est facultativement substitué avec un groupe hydroxy, alkyle en C₁ à C₆, hydroxyalkyle en C₁ à C₆, aminocarbonyle (alkyle en C₁ à C₆), alcényle en C₂ à C₆, alkylsulfonyle en C₁ à C₆, trifluorométhylsulfonyle, alcénylsulfonyle en C₂ à C₆, (alkoxy en C₁ à C₆) carbonyle, (alkyle en C₁ à C₆)carbonyle, (alcényle en C₂ à C₆)oxycarbonyle ou (alcényle en C₂ à C₆)carbonyle et est en outre facultativement substitué avec un substituant alkyle en C₁ à C₆, hydroxyalkyle en C₁ à C₆, aminocarbonyl(alkyle en C₁ à C₆) ou alcényle en C₂ à C₆ ; cyano ; tétrazolyle ; 2-oxo-oxazolidinyle facultativement substitué avec un substituant R¹⁰; 3-hydroxy-3-cyclobutène-1,2-dione-4-yl ; 2,4-thiazolidinedione-5-yle ; tétrazol-5-ylaminocarbonyle ; 1,2,4-triazol-5-yle facultativement substitué avec un substituant R¹⁰ ; ou 5-oxo-1,2,4-oxadiazol-3-yle ; ou
alkyle en C₁ à C₄ ou éthényle facultativement substitué avec n'importe lequel des groupes énumérés ci-dessus pour R³ et/ou 0 à 2 groupes R¹² choisis indépendamment entre des groupes:
halogéno ; alkylthio en C₁ à C₆ ; trifluorométhyle ; (alkoxy en C₁ à C₆)carbonyle; (alkyle en C₁ à C₆) carbonyle ; (alcényle en C₂ à C₆)oxycarbonyle ; (alcényle en C₂ à C6) carbonyle ; hydroxy facultativement substitué avec un groupe alkyle en C₁ à C₆, alcényle en C₂ à C₆, (alkoxy en C₁ à C₆) carbonyle, (alkyle en C₁ à C₆) carbonyle, (alcényle en C₂ à C₆) oxycarbonyle, (alcényle en C₂ à C₆) carbonyle ou aminocarbonyle dans lequel le groupe amino est facultativement substitué avec un substituant alkyle en C₁ à C₆, alcényle en C₂ à C₆, (alkyle en C₁ à C₆) carbonyle ou (alcényle en C₂ à C₆)carbonyle ; amino facultativement mono- ou disubstitué avec un substituant (alkoxy en C₁ à C₆)carbonyle, (alkyle en C₁ à C₆)carbonyle, (alcényle en C₂ à C₆)oxycarbonyle, (alcényle en C₂ à C₆) carbonyle, alkyle en C₁ à C₆, alcényle en C₂ à C₆, alkylsulfonyle en C₁ à C₆, alcénylsulfonyle en C₂ à C₆ ou aminocarbonyle dans lequel le groupe amino est facultativement substitué avec un substituant alkyle en C₁ à C₆ ou alcényle en C₂ à C₆ ; aminocarbonyle dans lequel le groupe amino est facultativement substitué avec un substituant alkyle en C₁ à C₆, hydroxyalkyle en C₁ à C₆, aminocarbonyl-(alkyle en C₁ à C₆), alcényle en C₂ à C₆, (alkoxy en C₁ à C₆) carbonyle, (alkyle en C₁ à C₆) carbonyle, (alcényle en C₂ à C₆)oxycarbonyle ou (alcényle en C₂ à C₆)carbonyle et est en outre facultativement substitué avec un substituant alkyle en C₁ à C₆, hydroxyalkyle en C₁ à C₆, aminocarbonyle(alkyle en C₁ à C₆) ou alcényle en C₂ ou C₆ ; oxo ; alkylsulfonyle en C₁ à C₆ ; alcénylsulfonyle en C₂ à C₆ ; ou aminosulfonyle en C₁ à C₆ dans lequel le groupe amino est facultativement substitué avec un substituant alkyle en C₁ à C₆ ou alcényle en C₂ à C₆ ;
en outre, lorsque R³ est disubstitué avec un substituant à fonction hydroxy ou amino et un substituant à fonction carboxy, ceux-ci peuvent former facultativement conjointement une liaison ester ou amide cyclique, respectivement ;
R¹⁰ est choisi entre des groupes alkyle en C₁ à C₄, alcényle en C₂ à C₄ et aryle, dont n'importe lequel peut être facultativement substitué avec un groupe R¹² répondant à la définition précitée ; carboxy ; et aminocarbonyle dans lequel le groupe amino est facultativement substitué avec un substituant hydroxy, alkyle en C₁ à C₆, alcényle en C₂ à C₆, alkylsulfonyle en C₁ à C₆, trifluorométhylsulfonyle, alcénylsulfonyle en C₂ à C₆, (alkoxy en C₁ à C₆) carbonyle, (alkyle en C₁ à C₆)carbonyle, (alcényle en C₂ à C₆)oxycarbonyle ou (alcényle en C₂ à C₆)carbonyle ;
R⁴ représente un groupe -U-V-R⁵, dans lequel R⁵ représente un système de noyau carbocyclique ou hétérocyclique bicyclique (A) facultativement substitué : 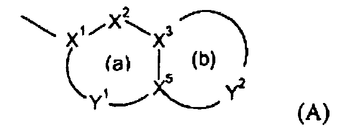 contenant jusqu'à quatre hétéroatomes dans chaque noyau, dans lequel
au moins un des noyaux (a) et (b) est aromatique ;
x¹ représente C ou N lorsqu'il fait partie d'un noyau aromatique ou un groupe CR¹⁴ ou N lorsqu'il fait partie d'un noyau non aromatique ;
X² représente N, un groupe NR¹³, O, S(O)ₓ, CO ou CR¹⁴ lorsqu'il fait partie d'un noyau aromatique ou non aromatique ou peut représenter en outre un groupe CR¹⁴R¹⁵, lorsqu'il fait partie d'un noyau non aromatique ;
X³ et X⁵ représentent indépendamment N ou C ;
Y¹ représente un groupe de liaison de 0 à 4 atomes dont chaque atome est choisi indépendamment entre N, NR¹³, O, S(O)ₓ, CO et CR¹⁴, lorsqu'il fait partie d'un noyau aromatique ou non aromatique ou peut représenter en outre un groupe CR¹⁴R¹⁵ lorsqu'il fait partie d'un noyau non aromatique,
Y² représente un groupe de liaison de 2 à 6 atomes, chaque atome de Y² étant choisi indépendamment entre N, NR¹³, O, S(O)ₓ, CO et CR¹⁴, lorsqu'il fait partie d'un noyau aromatique ou non aromatique ou peut représenter en outre un groupe CR¹⁴R¹⁵ lorsqu'il fait partie d'un noyau non aromatique ;
chacun des groupes R¹⁴ et R¹⁵ est choisi indépendamment entre H ; des groupes alkylthio en C₁ à C₄ ; halogéno ; carboxy (alkyle en C₁ à C₄) ; halogénalkoxy en C₁ à C₄ ; halogénalkyle en C₁ à C₄ ; alkyle en C₁ à C₄ ; alcényle en C₂ à C₄ ; (alkoxy en C₁ à C₄)carbonyle ; formyle ; (alkyle en C₁ à C₄)carbonyle ; (alcényle en C₂ à C₄)oxycarbonyle ; (alcényle en C₂ à C₄)carbonyle ; (alkyle en C₁ à C₄) - carbonyloxy ; (alkoxy en C₁ à C₄) carbonyle (alkyle en C₁ à C₄) ; hydroxy ; hydroxyalkyle en C₁ à C₄ ; mercapto (alkyle en C₁ à C₄) ; alkoxy en C₁ à C₄ ; nitro; cyano; carboxy ; amino ou aminocarbonyle facultativement substitués comme pour les substituants correspondants de R³ ; alkylsulfonyle en C₁ à C₄, alcénylsulfonyle en C₂ à C₄ ; ou aminosulfonyle dans lequel le groupe amino est facultativement substitué avec un substituant alkyle en C₁ à C₄ ou alcényle en C₂ à C₄ ; aryle ; aryl(alkyle en C₁ à C₄) ; et aryl(alkoxy en C₁ à C₄) ;
chaque groupe R¹³ représente indépendamment H ; un groupe trifluorométhyle ; alkyle en C₁ à C₄ facultativement substitué avec un substituant hydroxy, alkoxy en C₁ à C₆, alkylthio en C₁ à C₆, halogéno ou trifluorométhyle ; alcényle en C₂ à C₄ ; aryle ; aryl(alkyle en C₁ à C₄) ; (alkoxy en C₁ à C₄) carbonyle ; (alkyle en C₁ à C₄) carbonyle ; formyle ; alkylsulfonyle en C₁ à C₆ ; ou aminocarbonyle dans lequel le groupe amino est facultativement substitué avec un substituant (alkoxy en C₁ à C₄)carbonyle ; (alkyle en C₁ à C₄) carbonyle ; (alcényle en C₂ à C₄) oxycarbonyle ; (alcényle en C₂ à C₄) carbonyle ; alkyle en C₁ à C₄ ou alcényle en C₂ à C₄ et facultativement substitué en outre avec un substituant alkyle en C₁ à C₄ ou alcényle en C₂ à C₄ ;
U est choisi entre des groupes CO, SO₂ et CH₂ et V représente un groupe CR¹⁷R¹⁸ ou U représente un groupe CH₂ et V représente un groupe CO ou SO₂
R¹⁷ et R¹⁸ sont choisis indépendamment entre l'hydrogène ; un groupe hydroxy facultativement substitué avec un substituant alkyle en C₁ à C₆, alcényle en C₂ à C₆, (alkoxy en C₁ à C₆)carbonyle, (alkyle en C₁ à C₆) carbonyle, (alcényle en C₂ à C₆)oxycarbonyle, (alcényle en C₂ à C₆)carbonyle ou aminocarbonyle dans lequel le groupe amino est facultativement substitué avec un substituant alkyle en C₁ à C₆, alcényle en C₂ à C₆, (alkyle en C₁ à C₆) carbonyle ou (alcényle en C₂ à C₆) carbonyle ; et un groupe amino facultativement mono- ou disubstitué avec un substituant (alkoxy en C₁ à C₆) carbonyle, (alkyle en C₁ à C₆) carbonyle, (alcényle en C₂ à C₆)oxycarbonyle, (alcényle en C₂ à C₆) carbonyle, alkyle en C₁ à C₆, alcényle en C₂ à C₆, alkylsulfonyle en C₁ à C₆, alcénylsulfonyle en C₂ à C₆ ou aminocarbonyle dans lequel le groupe amino est facultativement substitué avec un substituant alkyle en C₁ à C₆ ou alcényle en C₂ à C₆ ;
n est égal à 0 et AB représente un groupe NR¹¹CO, CO-CR⁸R⁹, CR⁶R⁷-CO, NR¹¹SO₂, CR⁶R⁷-SO₂ ou CR⁶R⁷-CR⁸R⁹, sous réserve que R⁸ et R⁹ ne représentent pas des groupes hydroxy ou amino facultativement substitués et R⁶ et R⁸ ne représentent pas une liaison ;
ou bien n est égal à 1 et AB représente un groupe NR¹¹CO, CO-CR⁸R⁹, CR⁶R⁷-CO, NR¹¹SO₂, CONR¹¹, CR⁶R⁷-CR⁸R⁹, O-CR⁸R⁹ ou NR¹¹-CR⁸R⁹ ;
et dans laquelle :
chacun des groupes R⁶, R⁷, R⁸ et R⁹ est choisi indépendamment entre : H ; des groupes alkoxy en C₁ à C₆ ; alkylthio en C₁ à C₆ ; halogéno ; trifluorométhyle ; azido ; alkyle en C₁ à C₆ ; alcényle en C₂ à C₆ ; (alkoxy en C₁ à C₆) carbonyle ; (alkyle en C₁ à C₆) carbonyle ; (alcényle en C₂ à C₆) oxycarbonyle ; (alcényle en C₂ à C₆) carbonyle ; hydroxy, amino ou aminocarbonyle facultativement substitué comme pour les substituants correspondants dans R³ ; alkylsulfonyle en C₁ à C₆ ; alcénylsulfonyle en C₂ à C₆ ; ou aminosulfonyle en C₁ à C₆ dans lequel le groupe amino est facultativement substitué avec un substituant alkyle en C₁ à C₆ ou alcényle en C₂ à C₆ ;
ou bien R⁶ et R⁸, conjointement, représentent une liaison et R⁷ et R⁹ répondent aux définitions précitées ;
et chaque groupe R¹¹ représente indépendamment H ; un groupe trifluorométhyle, alkyle en C₁ à C₆ ; alcényle en C₂ à C₆ ; (alkoxy en C₁ à C₆) carbonyle ; (alkyle en C₁ à C₆)-carbonyle ; ou aminocarbonyle dans lequel le groupe amino est facultativement substitué avec un substituant (alkoxy en C₁ à C₆)carbonyle ; (alkyle en C₁ à C₆)carbonyle ; (alcényle en C₂ à C₆)oxycarbonyle ; (alcényle en C₂ à C₆) carbonyle ; alkyle en C₁ à C₆ ou alcényle en C₂ à C₆ et est facultativement substitué en outre avec un substituant alkyle en C₁ à C₆ ou alcényle en C₂ à C₆ ;
ou bien dans laquelle un des groupes R³ et R⁶, R⁷, R⁸ ou R⁹ contient un groupe carboxy et l'autre contient un groupe hydroxy ou amino ; ils peuvent conjointement former une liaison ester ou amide cyclique ;
où
le terme "hétérocyclique" désigne un noyau aromatique ou non aromatique, unique ou condensé, contenant dans chaque noyau jusqu'à quatre hétéroatomes choisis entre l'oxygène, l'azote et le soufre, et ayant 4 à 7 atomes dans le noyau, noyaux qui peuvent être non substitués ou C-substitués avec jusqu'à trois groupes choisis entre des groupes alkylthio en C₁ à C₄ ; halogéno ; carboxy (alkyle en C₁ à C₄) ; halogénalkoxy en C₁ à C₄ ; halogénalkyle en C₁ à C₄ ; alkyle en C₁ à C₄ ; alcényle en C₂ à C₄ ; alkoxy en C₁ à C₄)carbonyle ; formyle ; (alkyle en C₁ à C₄)carbonyle ; (alcényle en C₂ à C₄)oxycarbonyle ; (alcényle en C₂ à C₄) carbonyle ; (alkyle en C₁ à C₄) carbonyloxy ; (alkoxy en C₁ à C₄) carbonyle (alkyle en C₁ à C₄) ; hydroxy ; hydroxyalkyle en C₁ à C₄ ; mercapto (alkyle en C₁ à C₄) ; alkoxy en C₁ à C₄ ; nitro ; cyano ; carboxy ; amino ou aminocarbonyle facultativement substitués comme pour les substituants correspondants de R³ ; alkylsulfonyle en C₁ à C₄ ; alcénylsulfonyle en C₂ à C₄ ; ou aminosulfonyle dans lequel le groupe amino est facultativement substitué avec un substituant alkyle en C₁ à C₄ ou alcényle en C₂ à C₄ ; aryle facultativement substitué, aryl(alkyle en C₁ à C₄) ou aryl(alkoxy en C₁ à C₄) et oxo, et où n'importe quel groupe amino faisant partie d'un noyau hétérocyclique non aromatique unique ou condensé répondant à la définition précitée est facultativement substitué avec un substituant trifluorométhyle ; alkyle en C₁ à C₄ facultativement substitué avec un substituant hydroxy, alkoxy en C₁ à C₆, alkylthio en C₁ à C₆, halogéno ou trifluorométhyle ; alcényle en C₂ à C₄ ; aryle ; aryl (alkyle en C₁ à C₄) ; (alkoxy en C₁ à C₄)carbonyle ; (alkyle en C₁ à C₄)carbonyle ; formyle ; alkylsulfonyle en C₁ à C₆ ; ou aminocarbonyle dans lequel le groupe amino est facultativement substitué avec un substituant (alkoxy en C₁ à C₄)carbonyle, (alkyle en C₁ à C₄)carbonyle, (alcényle en C₂ à C₄)oxycarbonyle, (alcényle en C₂ à C₄)carbonyle, alkyle en C₁ à C₄ ou alcényle en C₂ à C₄ et est facultativement substitué en outre avec un substituant alkyle en C₁ à C₄ ou alcényle en C₂ à C₄ ;
le terme "aryle" désigne un groupe phényle ou naphtyle facultativement substitué avec jusqu'à cinq groupes choisis entre des groupes alkylthio en C₁ à C₄ ; halogéno ; carboxy (alkyle en C₁ à C₄) ; halogénalkoxy en C₁ à C₄ ; halogénalkyle en C₁ à C₄ ; alkyle en C₁ à C₄ ; alcényle en C₂ à C₄ ; (alkoxy en C₁ à C₄) carbonyle ; formyle ; (alkyle en C₁ à C₄)carbonyle ; (alcényle en C₂ à C₄)oxycarbonyle ; (alcényle en C₂ à C₄)carbonyle ; (alkyle en C₁ à C₄)carbonyloxy ; (alkoxy en C₁ à C₄) carbonyle (alkyle en C₁ à C₄) ; hydroxy ; hydroxyalkyle en C₁ à C₄ ; mercapto (alkyle en C₁ à C₄) ; alkoxy en C₁ à C₄ ; nitro ; cyano ; carboxy ; amino ou aminocarbonyle facultativement substitué comme pour les substituants correspondants de R³ ; alkylsulfonyle en C₁ à C₄, alcénylsulfonyle en C₂ à C₄; ou aminosulfonyle dans lequel le groupe amino est facultativement substitué avec un substituant alkyle en C₁ à C₄ ou alcényle en C₂ à C₄ ; phényle ; phényl(alkyle en C₁ à C₄) et phényl (alkoxy en C₁ à C₄) ;
le terme "acyle" désigne un groupe (alkoxy en C₁ à C₆)carbonyle, formyle ou (alkyle en C₁ à C₆)carbonyle.

2. Composé suivant la revendication 1, dans lequel Z⁵ représente un groupe CH ou N, Z³ représente un groupe CH ou CF et Z¹, Z² et Z⁴ représentent chacun un groupe CH, ou bien Z¹ représente N, Z³ représente un groupe CH ou CF et Z², Z⁴ et Z⁵ représentent chacun un groupe CH.

3. Composé suivant l'une quelconque des revendications précédentes, dans lequel R¹ représente un groupe méthoxy et R^{1a} représente H ou bien, lorsque Z³ représente un groupe CR^{1a}, il peut représenter un groupe C-F.

4. Composé suivant l'une quelconque des revendications précédentes, dans lequel R³ représente l'hydrogène ; un groupe CONH₂ ; 1-hydroxyalkyle ; CH₂CO₂H ; CH₂CONH₂ ; -CONHCH₂CONH₂ ; 1,2-dihydroxyalkyle ; CH₂CN ; 2-oxo-oxazolidine-5-yle ou 2-oxo-oxazolidine-5-yl (alkyle en C₁ à C₄).

5. Composé suivant l'une quelconque des revendications précédentes, dans lequel n est égal à 0 et soit A représente un groupe CHOH et B représente un groupe CH₂, soit A représente NH et B représente un groupe CO.

6. Composé suivant l'une quelconque des revendications précédentes, dans lequel le groupe -U-V- représente un groupe -(CH₂)₂-.

7. Composé suivant l'une quelconque des revendications précédentes, dans lequel R⁵ représente un noyau hétérocyclique aromatique (A) ayant 8 à 11 atomes dans le noyau comprenant 2 à 4 hétéroatomes dont au moins l'un est un atome de N ou un groupe NR¹³ ou bien le noyau hétérocyclique (A) possède un noyau (a) aromatique et un noyau (b) non aromatique et Y² possède 3 à 5 atomes, y compris NR¹³, 0 et S liés à X⁵ et NHCO lié par l'intermédiaire de N à X³, ou O ou un groupe NH lié à X³.

8. Composé suivant la revendication 1, choisi entre :
2-(2-{4-[(R)-2-hydroxy-2-(6-méthoxy-quinoléine-4-yl)-éthyl]pipérazine-1-yl}-éthyl)-isoindole-1,3-dione ;
(R)-2-{4-[2-(4-fluoro-1H-benzoimidazole-2-yl)-éthyl]-pipérazine-1-yl}-1-(6-méthoxy-quinoléine-4-yl)-éthanol ;
(6-méthoxy-[1,5]naphtyridine-4-yl)-amide d'acide 4-[2-(4-fluoro-1H-benzoimidazole-2-yl)-éthyl]-pipérazine-1-carboxylique ;
(R)-2-{4-[2-(5-fluoro-1H-benzoimidazole-2-yl)-éthyl]-pipérazine-1-yl}-1-(6-méthoxy-quinoléine-4-yl)-éthanol ;
(6-méthoxy-[1,5]naphtyridine-4-yl)-amide d'acide 4-[2-(2,3-dihydro-benzo-[1,4]dioxine-6-yl)-éthyl]-pipérazine-1-carboxylique ;
(6-méthoxy-[1,5]naphtyridine-4-yl)-amide d'acide 4-(2-benzo[1,3]dioxo-5-yl-éthyl)-pipérazine-1-carboxylique ;
(6-méthoxy-[1,5]naphtyridine-4-yl)-amide d'acide 4-[2-(3-oxo-3,4-dihydro-2H-benzo[1,4]thiazine-6-yl)-éthyl]-pipérazine-1-carboxylique ;
(6-méthoxy-[1,5]aphtyridine-4-yl)-amide d'acide 4-(2-quinoxaline-2-yl-éthyl)-pipérazine-1-carboxylique ;
(6-méthoxy-[1,5]phtyridine-4-yl)-amide d'acide 4-(2-benzo[1,2,5]thiadiazole-5-yl-éthyl)-pipérazine-1-carboxylique ;
(6-méthoxy-[1,5]naphtyridine-4-yl)-amide d'acide 4-(2-oxo-2-quinoxaline-2-yl-éthyl)-pipérazine-1-carboxylique ;
(6-méthoxy-[1,5]naphtyridine-4-yl)-amide d'acide 4-(2-hydroxy-2-quinoxaline-2-yl-éthyl)-pipérazine-1-carboxylique ;
(6-méthoxy-[1,5]naphtyridine-4-yl)-amide d'acide 4-[2-oxo-2-(3-oxo-3,4-dihydro-2H-benzo[1,4]oxazine-6-yl)-éthyl]-pipérazine-1-carboxylique ; et
(6-méthoxy-[1,5]naphtyridine-4-yl)-amide d'acide 4-[2-hydroxy-2-(3-oxo-3,4-dihydro-2H-benzo[1,4]oxazine-6-yl)-éthyl]-pipérazine-1-carboxylique
ou un de ses dérivés pharmaceutiquement acceptables.

9. Utilisation d'un composé suivant la revendication 1, dans la production d'un médicament destiné à être utilisé dans le traitement d'infections bactériennes chez des mammifères.

10. Composition pharmaceutique comprenant un composé suivant la revendication 1 et un support pharmaceutiquement acceptable.

11. Procédé pour la préparation d'un composé suivant la revendication 1, procédé qui comprend la réaction d'un composé de formule (IV) avec un composé de formule (V) : 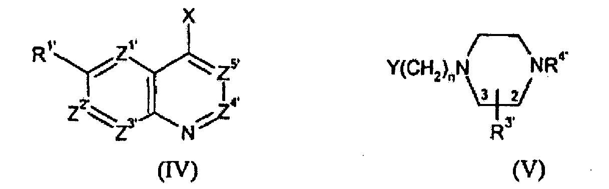 formules dans lesquelles n est tel que défini pour la formule (I) ; Z^{1'}, Z^{2'}, Z^{3'}, Z^{4'}, Z^{5'}, R^{1'}, R^{3'} et R^{4'} représentent des groupes Z¹, Z², Z³, Z⁴, Z⁵, R¹, R³ et R⁴ tels que définis pour la formule (I) ou des groupes pouvant être convertis en ceux-ci ; et X et Y peuvent représenter les associations suivantes :
(i) X représente un groupe A'-COW, Y représente H et n est égal à 0 ;
(ii) X représente un groupe CR⁶=CR⁸R⁹, Y représente H et n est égal à 0 ;
(iii) X représente un groupe oxiranne, Y représente H et n est égal à 0 ;
(iv) X représente un groupe N=C=O, Y représente H et n est égal à 0 ;
(v) un des groupes X et Y représente un groupe CO₂R^{y} et l'autre représente un groupe CH₂CO₂R^{x} ;
(vi) X représente un groupe CHR⁶R⁷ et Y représente un groupe C(=O)R⁸;
(vii) X représente un groupe CR⁷=PR^{z}₃ et Y représente un groupe C(=O)R⁹ et n est égal à 1 ;
(viii) X représente un groupe C(=O)R⁷ et Y représente un groupe CR⁹=PR^{z}₃ et n est égal à 1 ;
(ix) Y représente un groupe COW et X représente un groupe NHR^{11'} ou NR^{11'}COW et n est égal à 0 ou 1 ou, lorsque n est égal à 1, X représente un groupe COW et Y représente un groupe NHR^{11'} ou NR^{11'}COW ;
(x) X représente un groupe NHR^{11'} et Y représente un groupe C(=O)R⁸ et n est égal à 1 ;
(xi) X représente un groupe NHR^{11'} et Y représente un groupe CR⁸R⁹W et n est égal à 1 ;
(xii) X représente un groupe NR^{11'}COCH₂W ou NR^{11'}SO₂CH₂W et Y représente H et n est égal à 0 ;
(xiii) X représente un groupe CR⁶R⁷SO₂W et Y représente H et n est égal à 0 ;
(xiv) X représente un groupe W ou OH et Y représente un groupe CH₂OH et n est égal à 1 ;
(xv) X représente un groupe NHR^{11'} et Y représente un groupe SO₂W ou bien X représente un groupe NR^{11'}SO₂W et Y représente H et n est égal à 0 ;
où W représente un groupe partant, par exemple halogéno ou imidazolyle ; R^{x} et R^{y} représentent des groupes alkyle en C₁ à C₆ ; R^{z} représente un groupe aryle ou alkyle en C₁ à C₆ ; A' et NR^{11'} représentent des groupes A et NR¹¹ de la manière définie pour la formule (I), ou des groupes pouvant être convertis en ceux-ci ; et l'oxiranne répond à la formule : 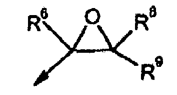 dans laquelle R⁶, R⁸ et R⁹ sont tels que définis pour la formule (I) ;
et ensuite, facultativement ou de manière nécessaire, la conversion de A', Z^{1'}, Z^{2'}, Z^{3'}, Z^{4'}, Z^{5'}, R^{1'}, R^{3'}, R^{4'} et NR^{11'} en des groupes A, Z¹, Z², Z³, Z⁴, Z⁵, R¹, R³, R^{4'} et NR¹¹ ; la conversion de A-B en un autre groupe A-B, l'inter-conversion de R¹, R³ et/ou R⁴ et/ou la formation d'un sel et/ou N-oxyde pharmaceutiquement acceptable du composé obtenu.
